(19) **Europäisches Patentamt / European Patent Office / Office européen des brevets**

(11) **EP 4 215 203 A1**

(12) **EUROPEAN PATENT APPLICATION**
published in accordance with Art. 153(4) EPC

(43) Date of publication:
**26.07.2023 Bulletin 2023/30**

(21) Application number: **21868189.8**

(22) Date of filing: **24.06.2021**

(51) International Patent Classification (IPC):
*A61K 36/88* (2006.01)     *A61K 36/734* (2006.01)
*A61K 36/704* (2006.01)     *A61K 36/535* (2006.01)
*A61K 36/484* (2006.01)     *A61K 36/481* (2006.01)
*A61K 36/35* (2006.01)     *A61K 36/34* (2006.01)
*A61K 36/28* (2006.01)     *A61K 36/71* (2006.01)
*A61P 11/04* (2006.01)     *A61P 31/14* (2006.01)
*A61P 11/10* (2006.01)     *A61P 11/14* (2006.01)

(86) International application number:
**PCT/CN2021/102174**

(87) International publication number:
**WO 2022/057360 (24.03.2022 Gazette 2022/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.09.2020 CN 202010989020**

(71) Applicant: **Tianjin University Of Traditional Chinese Medicine**
**Tianjin 300193 (CN)**

(72) Inventors:
• **ZHANG, Boli**
  **Tianjin 300193 (CN)**
• **ZHANG, Junhua**
  **Tianjin 300193 (CN)**
• **SONG, Xinbo**
  **Tianjin 300193 (CN)**
• **ZHANG, Han**
  **Tianjin 300193 (CN)**

• **MIAO, Lin**
  **Tianjin 300193 (CN)**
• **ZHOU, Kun**
  **Tianjin 300193 (CN)**
• **REN, Ming**
  **Tianjin 300193 (CN)**
• **LIU, Erwei**
  **Tianjin 300193 (CN)**
• **WANG, Yuefei**
  **Tianjin 300193 (CN)**
• **ZHENG, Wenke**
  **Tianjin 300193 (CN)**
• **YANG, Fengwen**
  **Tianjin 300193 (CN)**

(74) Representative: **Casalonga**
**Casalonga & Partners**
**Bayerstraße 71/73**
**80335 München (DE)**

Remarks:
The complete document including Reference Table(s) and the Sequence Listing(s) can be downloaded from the EPO website

(54) **PHARMACEUTICAL COMPOSITION FOR USE PREVENTING AND CURING RESPIRATORY DISEASES IN WINTER**

(57)     The present invention relates to a pharmaceutical composition for use preventing and curing respiratory diseases in winter. The composition comprises burdock seeds, blackberry lily, Platycodon grandiflorus, radix paeoniae rubra, Perilla frutescens, honeysuckle, fructus crataegi, Astragalus membranaceus, Reynoutria japonica, Glycyrrhiza uralensis, and non-essential fermented tea. The present invention also relates to a pharmaceutical preparation use of the pharmaceutical composition. The pharmaceutical composition can be used for nourishing Qi, moistening lungs, clearing throats, relieving sore throats and/or decontaminating, and can be used for preparing a pharmaceutical for treating and/or preventing pharyngitis.

## Description

## Technical Field

The invention belongs to the field of traditional Chinese medicines, and it relates to a traditional Chinese medicine composition for preventing or treating respiratory diseases in winter and a formulation thereof, in particular a tea formulation. The invention further relates to a method of using said pharmaceutical composition and use thereof in the manufacture of medicaments.

## Background Art

Winter is a season when respiratory diseases frequently occur, and pharyngitis is one common symptom of respiratory diseases. The pharyngitis has the characteristics of repeated attack, stubborn symptoms, prolonged disease course and the like, which produce great adverse impacts on daily life, study and work of patients and causes great trauma to physical and mental health of the patients. In clinic, the treatment of pharyngitis is mainly based on the use of antiviral medicaments and antibacterial medicaments, whereas sometimes, they cannot achieve satisfactory therapeutic effects and some serious side effects are also involved. For the treatment of the pharyngitis, traditional Chinese medicines in China more focus on starting from the overall of diseases and emphasizing dialectical treatments, and the achieved therapeutic effects are often more satisfactory whilst fewer side effects are produced. However, traditional Chinese medicine formulations currently applied in clinic are relatively limited, and no traditional Chinese medicine formulation for preventing or treating pharyngitis in winter, which can be conveniently used and achieve significantly effects, is reported.

Herb tea, i.e., a tea formulation, refers to an oral formulation prepared by mixing decoction pieces or extracts with tea leaves or other adjuvants, and it may be classified into a block tea formulation, a bagged tea formulation and a decocted tea formulation, being an important member of the traditional Chinese medicinal treasury. The bagged tea formulation refers to a tea formulation prepared by charging into bags tea leaves, coarse decoction piece powder or a part of coarse decoction piece powder which absorb extractive solutions and then dried, wherein those charged into tea bags for drinking are also referred as tea bag formulations. The tea bag formulation has the advantages of large doses, small volumes, convenient taking and quick dissolution, and it reserves the advantages and characteristics of traditional decoctions in a maximum level and more conform to public habits and customs. The formulation is increasingly developed into one of popular traditional Chinese medicinal dosages. In winter, taking traditional Chinese medicine tea formulations can achieve the beneficial effects of early intervention and early prevention.

Therefore, there is a need that is still not met for the means of preventing or treating respiratory diseases, e.g., pharyngitis, in winter, improving life qualities of patients, and/or preventing recurrences of the pharyngitis

## Disclosure of the Invention

It is one object of the invention is to provide a traditional Chinese medicine composition and a traditional Chinese medicine formulation for preventing and/or treating respiratory diseases in winter.

It is another object of the present invention is to provide use of traditional Chinese herbal medicines in the manufacture of a traditional Chinese medicine composition or a traditional Chinese medicine formulation for preventing and/or treating respiratory diseases in winter.

It is a further object of the present invention to provide a method for preventing and/or treating respiratory diseases in winter, comprising administering an effective amount of a traditional Chinese medicine composition or a traditional Chinese medicine formulation to a subject in need.

The inventors, through intensive research and creative work, obtain a pharmaceutical composition. The inventors surprisingly find that the pharmaceutical composition can nourish yin and relieve sore throat, and have the efficacies of nourishing Qi, moistening lungs, clearing throats, relieving sore throats, and decontaminating, and it can effectively treat and/or prevent respiratory diseases in winter such as pharyngitis and the like, and effectively eliminate phlegm and/or relieve cough. On this basis, the inventors have finished the invention.

The invention may be described in various aspects, and the inventions described in these aspects and any forms thereof are, independently but also interrelatedly, combined with each other to form the subject matter of the invention.

In one aspect, the present invention provides a traditional Chinese medicine composition, a traditional Chinese medicine formulation or a tea bag formulation for treating and/or preventing respiratory diseases in winter.

The traditional Chinese medicine composition of the invention is composed of or prepared with the following traditional Chinese medicinal materials in medicinal forms:

Arctii Fructus, Belamcandae Rhizoma, Platycodonis Radix, Paeoniae Radix Rubra, Perillae folium, Lonicerae Japonicae Flos, Charred Hawthorn, Astragali Radix, Polygoni Cuspidati Rhizoma et Radix, Glycyrrhizae Radix et Rhizoma, and non-essential fermented tea.

The traditional Chinese medicine composition of the invention may be composed of or prepared with a mixture of decoction piece powders of the following traditional Chinese medicinal materials or a powder of a decoction piece mixture of the following traditional Chinese medicinal materials:

Arctii Fructus, Belamcandae Rhizoma, Platycodonis Radix, Paeoniae Radix Rubra, Perillae folium, Lonicerae Japonicae Flos, Charred Hawthorn, Astragali Radix, Polygoni Cuspidati Rhizoma et Radix, Glycyrrhizae Radix et Rhizoma, and non-essential fermented tea.

The traditional Chinese medicine composition of the invention may be also composed of the individual recipe granules of the following traditional Chinese medicinal materials:

Arctii Fructus, Belamcandae Rhizoma, Platycodonis Radix, Paeoniae Radix Rubra, Perillae folium, Lonicerae Japonicae Flos, Charred Hawthorn, Astragali Radix, Polygoni Cuspidati Rhizoma et Radix, Glycyrrhizae Radix et Rhizoma, and non-essential fermented tea.

The traditional Chinese medicine composition of the invention may be further composed of decoction piece extracts of the following traditional Chinese medicinal materials:

Arctii Fructus, Belamcandae Rhizoma, Platycodonis Radix, Paeoniae Radix Rubra, Perillae folium, Lonicerae Japonicae Flos, Charred Hawthorn, Astragali Radix, Polygoni Cuspidati Rhizoma et Radix, Glycyrrhizae Radix et Rhizoma, and non-essential fermented tea.

The traditional Chinese medicine formulation of the invention comprises the traditional Chinese medicine composition of the invention, and it may further comprise pharmaceutically acceptable adjuvants. In other words, the traditional Chinese medicine formulation of the invention comprises the following traditional Chinese medicinal materials or extracts thereof in medicinal forms: Arctii Fructus, Belamcandae Rhizoma, Platycodonis Radix, Paeoniae Radix Rubra, Perillae folium, Lonicerae Japonicae Flos, Charred Hawthorn, Astragali Radix, Polygoni Cuspidati Rhizoma et Radix, Glycyrrhizae Radix et Rhizoma, and non-essential fermented tea;

and pharmaceutically acceptable adjuvants.

The traditional Chinese medicine formulation of the invention may be formulated into an oral liquid or a decoction, comprising water extracts of the following traditional Chinese medicinal materials:

Arctii Fructus, Belamcandae Rhizoma, Platycodonis Radix, Paeoniae Radix Rubra, Perillae folium, Lonicerae Japonicae Flos, Charred Hawthorn, Astragali Radix, Polygoni Cuspidati Rhizoma et Radix, Glycyrrhizae Radix et Rhizoma, and non-essential fermented tea;
and pharmaceutically acceptable adjuvants.

The traditional Chinese medicine formulation of the invention can be formulated into a tea bag formulation, comprising decoction piece powders of the following traditional Chinese medicinal materials:

Arctii Fructus, Belamcandae Rhizoma, Platycodonis Radix, Paeoniae Radix Rubra, Perillae folium, Lonicerae Japonicae Flos, Charred Hawthorn, Astragali Radix, Polygoni Cuspidati Rhizoma et Radix, Glycyrrhizae Radix et Rhizoma and non-essential fermented tea;
and pharmaceutically acceptable adjuvants or acceptable additives in food or beverage.

The traditional Chinese medicine formulation of the invention may be formulated into granules, comprising decoction piece extracts of the following traditional Chinese medicinal materials:

Arctii Fructus, Belamcandae Rhizoma, Platycodonis Radix, Paeoniae Radix Rubra, Perillae folium, Lonicerae Japonicae Flos, Charred Hawthorn, Astragali Radix, Polygoni Cuspidati Rhizoma et Radix, Glycyrrhizae Radix et Rhizoma and non-essential fermented tea;
and pharmaceutically acceptable adjuvants.

The traditional Chinese medicine formulation of the invention may be formulated into pills, comprising decoction piece extracts of the following traditional Chinese medicinal materials:

Arctii Fructus, Belamcandae Rhizoma, Platycodonis Radix, Paeoniae Radix Rubra, Perillae folium, Lonicerae Japonicae Flos, Charred Hawthorn, Astragali Radix, Polygoni Cuspidati Rhizoma et Radix, Glycyrrhizae Radix et Rhizoma, and non-essential fermented tea;
and pharmaceutically acceptable adjuvants.

The traditional Chinese medicine formulation of the invention may be formulated into capsules, comprising decoction piece extracts of the following traditional Chinese medicinal materials:

Arctii Fructus, Belamcandae Rhizoma, Platycodonis Radix, Paeoniae Radix Rubra, Perillae folium, Lonicerae Japonicae Flos, Charred Hawthorn, Astragali Radix, Polygoni Cuspidati Rhizoma et Radix, Glycyrrhizae Radix et Rhizoma and non-essential fermented tea;
and pharmaceutically acceptable adjuvants.

The traditional Chinese medicine formulation of the invention may be formulated into syrups, comprising decoction piece extracts of the following traditional Chinese medicinal materials:

Arctii Fructus, Belamcandae Rhizoma, Platycodonis Radix, Paeoniae Radix Rubra, Perillae folium, Lonicerae Japonicae Flos, Charred Hawthorn, Astragali Radix, Polygoni Cuspidati Rhizoma et Radix, Glycyrrhizae Radix et Rhizoma and non-essential fermented tea;
and pharmaceutically acceptable adjuvants.

The traditional Chinese medicine formulation of the invention may be formulated into ointments, comprising decoction piece extracts of the following traditional Chinese medicinal materials:

Arctii Fructus, Belamcandae Rhizoma, Platycodonis Radix, Paeoniae Radix Rubra, Perillae folium, Lonicerae Japonicae Flos, Charred Hawthorn, Astragali Radix, Polygoni Cuspidati Rhizoma et Radix, Glycyrrhizae Radix et Rhizoma and non-essential fermented tea;
and pharmaceutically acceptable adjuvants.

The tea bag formulation of the invention comprises decoction piece powders of the following traditional Chinese medicinal materials:

Arctii Fructus, Belamcandae Rhizoma, Platycodonis Radix, Paeoniae Radix Rubra, Perillae folium, Lonicerae Japonicae Flos, Charred Hawthorn, Astragali Radix, Polygoni Cuspidati Rhizoma et Radix, Glycyrrhizae Radix et Rhizoma and non-essential fermented tea;
and pharmaceutically acceptable adjuvants or acceptable additives in food and beverage.

In the other aspect, the application provides use of traditional Chinese herbs in the manufacture of a traditional Chinese medicine composition, a traditional Chinese medicine formulation or a tea bag formulation for preventing and treating respiratory diseases in winter, the preventing and treating respiratory diseases including one, more or all of (1) nourishing Qi, moistening lungs, clearing throats, relieving sore throats, and/or decontaminating, (2) treating and preventing pharyngitis; (3) eliminating phlegm and (4) relieving cough, wherein the traditional Chinese medicine composition, the raditional Chinese medicine formulation or the tea bag formulation has the above-defined composition.
In a further aspect, the present invention provides a method for preventing and/or treating respiratory diseases in winter, comprising administering an effective amount of the traditional Chinese medicine composition, the traditional Chinese medicine formulation or the tea bag formulation of the invention to a subject in need, the preventing and/or treating respiratory diseases including one, more or all of (1) nourishing Qi, moistening lungs, clearing throats, relieving sore throats, and/or decontaminating, (2) treating and preventing pharyngitis; (3) eliminating phlegm and (4) relieving cough, and wherein the traditional Chinese medicine composition, the traditional Chinese medicine formulation or the tea bag formulation has the above-defined composition.
The traditional Chinese medicine composition, the traditional Chinese medicine formulation or the tea bag formulation of the invention can be used to produce technical effects as described in one, more or all of the following items (1) to (5):

(1) nourishing yin and relieving sore throat;
(2) nourishing Qi, moistening lungs, clearing throats, relieving sore throats, and decontaminating,
(3) effectively treating and preventing pharyngitis;
(4) effectively eliminating phlegm; and
(5) effectively relieving cough.

**Brief description of the drawings**

FIG. 1A: thin-layer identification results of Paeoniae Radix Rubra. (1) a Peoniflorin control, (2) an invention pharmaceutical composition, (3) a Paeoniae Radix Rubra-negative inventive pharmaceutical composition.

FIG. 1B: thin-layer identification results of Belamcandae Rhizoma. (1) an Irisflorentin control, (2) a Belamcandae Rhizoma control, (3) an inventive pharmaceutical composition, (4) a Belamcandae Rhizoma-negative inventive pharmaceutical composition.

FIG. 1C: thin-layer identification results of Polygoni Cuspidati Rhizoma et Radix. (1) a Polydatin control, (2) an inventive pharmaceutical composition.

Fig. 2A-2D: pathological sections of pharyngeal tissues of rats suffered from acute pharyngitis (HE×10), wherein Fig. 2A. a blank group; FIG. 2B. a model group; Fig. 2C. an amoxicillin group; Fig. 2D. an inventive pharmaceutical composition group.

FIG. 3: impacts of an inventive pharmaceutical composition sample on the TNF-$\alpha$ content in peripheral blood of rats suffered from acute pharyngitis. In comparison to the normal group, **P<0.01; in comparison to the model group, **P<0.01.

FIG. 4: impacts of an inventive pharmaceutical composition sample on the IL-1$\beta$ content in peripheral blood of rats suffered from acute pharyngitis. In comparison to the normal group, **P<0.01; in comparison to the model group, *P<0.05.

FIG. 5: impacts of an inventive pharmaceutical composition sample on the IL-6 content in peripheral blood of rats suffered from acute pharyngitis.

FIG. 6A: viability values ($\bar{x} \pm$ SD) (n=6) of THP-1 cells treated with an inventive pharmaceutical composition sample, detected by a CCK-8 method; **P<0.01 vs control, *P<0.001 vs control.

FIG. 6B: viability values ($\bar{x} \pm$ SD) (n=6) of THP-1 cells treated with an inventive pharmaceutical composition sample, detected by a LDH method; **P<0.01 vs control, *P<0.001 vs control.

FIG. 7A: inhibitions of an inventive pharmaceutical composition sample on the expression ($\bar{x} \pm$ SD) (n=3) of the inflammatory factor TNF-$\alpha$ in LPS-induced THP-1 cells; **P<0.01 vs control, ##P<0.01 vs LPS, #P<0.001 vs LPS.

FIG. 7B: inhibitions of an inventive pharmaceutical composition sample on the expression ($\bar{x} \pm$ SD) (n=3) of the inflammatory factor IP-10 in LPS-induced THP-1 cells; **P<0.01 vs control, ##P<0.01 vs LPS, #P<0.001 vs LPS.

FIG. 7C: inhibitions of an inventive pharmaceutical composition sample on the expression ($\bar{x} \pm$ SD) (n=3) of the inflammatory factor IKB$\alpha$ in LPS-induced THP-1 cells; **P<0.01 vs control, ##P<0.01 vs LPS, #P<0.001 vs LPS.

FIG. 7D: inhibitions of an inventive pharmaceutical composition sample on the expression ($\bar{x} \pm$ SD) (n=3) of the inflammatory factor IL-6 in LPS-induced THP-1 cells; **P<0.01 vs control, ##P<0.01 vs LPS, #P<0.001 vs LPS.

FIG. 7E: inhibitions of an inventive pharmaceutical composition sample on the expression ($\bar{x} \pm$ SD) (n=3) of the inflammatory factor IL-1$\beta$ in LPS-induced THP-1 cells; **P<0.01 vs control, ##P<0.01 vs LPS, #P<0.001 vs LPS.

FIG. 8A: impacts ($\bar{x} \pm$ SD) (n=18) of an inventive pharmaceutical composition sample on the viability of 293T cells, detected by a CCK-8 method; **P<0.01 vs control, *P<0.001 vs control.

FIG. 8B: impacts ($\bar{x} \pm$ SD) (n=18) of an inventive pharmaceutical composition sample on the viability of 293T cells, detected by a LDH method; **P<0.01 vs control, *P<0.001 vs control.

FIG. 9A: Impacts ($\bar{x} \pm$ SD) (n=18) of an inventive pharmaceutical composition sample with different concentrations on ARE luciferase activities of 293T cells. Note: **P<0.001 vs control, *P<0.05 vs control.

FIG. 9B: Impacts ($\bar{x} \pm$ SD) (n=18) of an inventive pharmaceutical composition sample with different concentrations on NF-$\kappa$B promoter luciferase activities of 293T cells. Note: **P<0.01 vs control, *P<0.05 vs control, ##P<0.01 vs model; #P<0.05 vs model.

**Best modes for carrying out the invention**

The previous text has summarized the invention in general aspects, and further, the invention will be described below in details by combining examples.

To accurately appreciate the terms used in the invention, the meanings of a part of the terms are specifically defined below. For the terms that are not specifically defined here, they have meanings that are commonly appreciated and accepted by those skilled in the art. If the meanings of some term defined here are not consistent with the meanings that are commonly appreciated and accepted by those skilled in the art, the meaning of the term should be based on the meanings as defined here.

The term "Arctii Fructus" used in the invention refers to dried ripe fruits of compositae, Arctium lappa L.

The term "Belamcandae Rhizoma" used in the invention refers to dried rootstalk of Iride, Belamcanda chinensis (L.) DC.

The term "Platycodonis Radix" used in the invention refers to dried roots of Campanulaceae, Platycodon grandiflorum (Jacq.) A. DC.

The term "Paeoniae Radix Rubra" used in the invention refers to dried roots of Ranunculaceae, Paeonia lactiflora Pall or Paeonia veitchii Lynch.

The term "Perillae folium" used in the invention refers to dried leaves (or with shoots) of Labiate, Perilla frutescens (L.) britt.

The term "Lonicerae Japonicae Flos" used in the invention refers to dried buds or buds with first bloomed flowers of Caprifoliaceae, Lonicera japonica Thunb.

The term "charred hawthorn" used in the invention refers to hawthorn products with scorched brown surfaces and yellow brown interiors prepared by processing hawthorns according to a single stir-drying method (General Rule 0213) recorded in Pharmacopoeia of the People's Republic of China. The "hawthorn" refers to dried ripe fruits of Rosaceae, Crataegus pinnattifolia Bge.var. major N.E.Br, or Crataegus pinnattifolia Bge.

The term "Astragali Radix" used in the invention refers to dried roots of Leguminosae, Astragalus membranaceus (Fisch.) Bge.var.mongholicus (Bge.) Hsiao or Astragalus membranaceus (Fisch.) Bge.

The term "Polygoni Cuspidati Rhizoma et Radix" used in the invention refers to dried rootstalk and roots of Polygonum, cuspidatum Sieb. et Zucc.

The term "Glycyrrhizae Radix et Rhizoma" used in the invention refers to dried roots and rootstalk of Leguminosae, Glycyrrhiza uralensis Fisch, Glycyrrhiza inflata Bat, or Glycyrrhiza glabra L.

The term "fermented tea" used in the invention refers to tea articles obtained by fermenting tea leaves, such as red tea, dark tea and oolong tea, and the "tea leaves" refer to leaves of Camellia sinensis.

The term "decoction piece powder of traditional Chinese medicine materials" used in the invention refers to an ingredient-full powder of traditional Chinese medicinal materials free of additives, which is processed by physical methods, such as coarse pulverizing, fine pulverizing, micronizing, physical wall-breaking pulverizing and the like.

The term "recipe granules" used in the invention refers to a single traditional Chinese medicine product prepared by extracting, concentrating, drying and other processes of traditional Chinese medicine decoction pieces according to a traditional decocting method of traditional Chinese medicine decoctions. The product retains the nature, taste and efficacies of traditional Chinese medicinal decoction pieces and has a stable and reliable quality, it may be applied in the prescription deployment of clinical diagnoses in traditional Chinese medical science and suited to requirements of treatments based on syndrome differentiations and prescription changes, and it has the advantages of no decoction and convenient taking.

The term "extract" used in the invention refers to a substance obtained by extracting said traditional Chinese medicinal materials in any forms (including traditional Chinese medicinal decoction pieces, traditional Chinese medicinal powder, such as micronized traditional Chinese medicinal powder) with a suitable pharmaceutically acceptable solvent such as water or an alcoholic aqueous solution, including specific effective ingredients and mixtures containing effective ingredients. The substance is suitable for rescues of patients in the decubation of severe diseases, e.g., Coronary Pneumonia, effectively preventing, treating or relieving sequelae or complications of infectious diseases or promoting function recoveries of damaged tissues, organs or systems caused by infectious diseases. Forms of the extract include, but are not limited to, solids, semisolids, solutions, suspensions, concentrates, ointments, and powders. Suitable water used in the invention for extracting traditional Chinese medicinal materials include various water which can be used for preparing traditional Chinese medicinal active extracts, e.g., medicinal water such as distilled water and deionized water.

The term "alcoholic aqueous solution" used in the invention refers to alcohol-containing aqueous solutions with suitable concentrations (e.g., low concentrations, particularly 10-50% v/v). Suitable alcohols include various alcohols, preferably ethanol. Under some conditions, an alcoholic aqueous solution with a concentration higher than 50% v/v can be also used.

The terms "patient" and "subject" used in the invention, interchangeably with each other, refer to mammals, particularly human being, suffering from respiratory diseases, e.g., upper respiratory diseases such as pharyngitis, laryngitis or pharyngolaryngitis.

The term "pharmaceutically acceptable adjuvants" used in the invention refers to any adjuvants conventionally used in the field of pharmaceutical formulations, as long as the adjuvants will not produce adverse reactions or impacts on intended qualities and therapeutic effects of the traditional Chinese medicine composition of the invention.

The term "acceptable additives in foods or beverages" used in the invention refers to any additives that can be added to food or beverages according to national laws and regulations and departmental regulations, such as flavoring agents, sweetening agents, materials used in both medicines and food, and the like.

The term "effective amount" used in the invention refers to an amount of the pharmaceutical composition of the invention that can prevent or treat said respiratory diseases or alleviate and/or relieve one or more symptoms of said respiratory diseases in a subject.

All numerical ranges disclosed in the application include the endpoints thereof and any sub-ranges therein that are not expressly listed.

The invention provides a pharmaceutical composition for preventing and/or treating respiratory diseases in winter, composed of the following traditional Chinese medicinal materials or extracts thereof in medicinal forms by weight parts:

2-4 parts by weight of Arctii Fructus,
1-3 parts by weight of Belamcandae Rhizoma,
1-3 parts by weight of Platycodonis Radi,
1-3 parts by weight of Paeoniae Radix Rubra,
1-3 parts by weight of Perillae folium,
2-4 parts by weight of Lonicerae Japonicae Flos,
1-3 parts by weight of Charred Hawthorn,
2-4 parts by weight of Astragali Radix,
2-4 parts by weight of Polygoni Cuspidati Rhizoma et Radix, and
0.5-1.5 parts by weight of Glycyrrhizae Radix et Rhizoma; as well as
0.5-8 parts by weight of non-essential fermented tea, e.g., red tea, black tea or oolong tea.

In some embodiments, the pharmaceutical composition is composed of the following traditional Chinese medicinal materials or extracts thereof in medicinal forms by weight parts:

2.5-3.5 parts by weight of Arctii Fructus,
1.5-2.5 parts by weight of Belamcandae Rhizoma,
1.5-2.5 parts by weight of Platycodonis Radi,
1.5-2.5 parts by weight of Paeoniae Radix Rubra,
1.5-2.5 parts by weight of Perillae folium,
2.5-3.5 parts by weight of Lonicerae Japonicae Flos,
1.5-2.5 parts by weight of Charred Hawthorn,
2.5-3.5 parts by weight of Astragali Radix,
2.5-3.5 parts by weight of Polygoni Cuspidati Rhizoma et Radix, and
0.8-1.2 parts by weight of Glycyrrhizae Radix et Rhizoma; as well as
2-6 parts by weight, preferably 3-5 parts by weight, of non-essential fermented tea, e.g., red tea, black tea or oolong tea.

In specific embodiments, the pharmaceutical composition is composed of the following traditional Chinese medicinal materials or extracts thereof in medicinal forms by weight parts:

3 parts by weight of Arctii Fructus,
2 parts by weight of Belamcandae Rhizoma,
2 parts by weight of Platycodonis Radi,
2 parts by weight of Paeoniae Radix Rubra,
2 parts by weight of Perillae folium,
3 parts by weight of Lonicerae Japonicae Flos,
2 parts by weight of Charred Hawthorn,
3 parts by weight of Astragali Radix,

3 parts by weight of Polygoni Cuspidati Rhizoma et Radix, and
1 part by weight of Glycyrrhizae Radix et Rhizoma.

In specific embodiments, the pharmaceutical composition is composed of the following traditional Chinese medicinal materials or extracts thereof in medicinal forms by weight parts:

3 parts by weight of Arctii Fructus,
2 parts by weight of Belamcandae Rhizoma,
2 parts by weight of Platycodonis Radi,
2 parts by weight of Paeoniae Radix Rubra,
2 parts by weight of Perillae folium,
3 parts by weight of Lonicerae Japonicae Flos,
2 parts by weight of Charred Hawthorn,
3 parts by weight of Astragali Radix,
3 parts by weight of Polygoni Cuspidati Rhizoma et Radix,
1 part by weight of Glycyrrhizae Radix et Rhizoma; and
3 parts by weight of red tea.

In specific embodiments, the pharmaceutical composition is composed of the following traditional Chinese medicinal materials or extracts thereof in medicinal forms by weight parts:

3 parts by weight of Arctii Fructus,
2 parts by weight of Belamcandae Rhizoma,
2 parts by weight of Platycodonis Radi,
2 parts by weight of Paeoniae Radix Rubra,
2 parts by weight of Perillae folium,
3 parts by weight of Lonicerae Japonicae Flos,
2 parts by weight of Charred Hawthorn,
3 parts by weight of Astragali Radix,
3 parts by weight of Polygoni Cuspidati Rhizoma et Radix,
1 part by weight of Glycyrrhizae Radix et Rhizoma; and
4 parts by weight of red tea.

In specific embodiments, the pharmaceutical composition is composed of the following traditional Chinese medicinal materials or extracts thereof in medicinal forms by weight parts:

3 parts by weight of Arctii Fructus,
2 parts by weight of Belamcandae Rhizoma,
2 parts by weight of Platycodonis Radi,
2 parts by weight of Paeoniae Radix Rubra,
2 parts by weight of Perillae folium,
3 parts by weight of Lonicerae Japonicae Flos,
2 parts by weight of Charred Hawthorn,
3 parts by weight of Astragali Radix,
3 parts by weight of Polygoni Cuspidati Rhizoma et Radix,
1 part by weight of Glycyrrhizae Radix et Rhizoma; and
5 parts by weight of red tea.

In preferred embodiments, the pharmaceutical composition is in a single dose form composed of the following traditional Chinese medicinal materials or extracts thereof in medicinal forms by weight parts:

3 g of stir-dried Arctii Fructus,
2 g of Belamcandae Rhizoma,
2 g of Platycodonis Radi,
2 g of Paeoniae Radix Rubra,
2 g of Perillae folium,
3 g of Lonicerae Japonicae Flos,
2 g of Charred Hawthorn,

3 g of Astragali Radix,
3 g of Polygoni Cuspidati Rhizoma et Radix, and
1 g of Glycyrrhizae Radix et Rhizoma.

In preferred embodiments, the pharmaceutical composition is in a single dose form composed of the following traditional Chinese medicinal materials or extracts thereof in medicinal forms by weight parts:

3 g of stir-dried Arctii Fructus,
2 g of Belamcandae Rhizoma,
2 g of Platycodonis Radi,
2 g of Paeoniae Radix Rubra,
2 g of Perillae folium,
3 g of Lonicerae Japonicae Flos,
2 g of Charred Hawthorn,
3 g of Astragali Radix,
3 g of Polygoni Cuspidati Rhizoma et Radix,
1 g of Glycyrrhizae Radix et Rhizoma, and
3 g of red tea.

In preferred embodiments, the pharmaceutical composition is in a single dose form composed of the following traditional Chinese medicinal materials or extracts thereof in medicinal forms by weight parts:

3 g of stir-dried Arctii Fructus,
2 g of Belamcandae Rhizoma,
2 g of Platycodonis Radi,
2 g of Paeoniae Radix Rubra,
2 g of Perillae folium,
3 g of Lonicerae Japonicae Flos,
2 g of Charred Hawthorn,
3 g of Astragali Radix,
3 g of Polygoni Cuspidati Rhizoma et Radix,
1 g of Glycyrrhizae Radix et Rhizoma, and
4 g of red tea.

In preferred embodiments, the pharmaceutical composition is in a single dose form composed of the following traditional Chinese medicinal materials or extracts thereof in medicinal forms by weight parts:

3 g of stir-dried Arctii Fructus,
2 g of Belamcandae Rhizoma,
2 g of Platycodonis Radi,
2 g of Paeoniae Radix Rubra,
2 g of Perillae folium,
3 g of Lonicerae Japonicae Flos,
2 g of Charred Hawthorn,
3 g of Astragali Radix,
3 g of Polygoni Cuspidati Rhizoma et Radix,
1 g of Glycyrrhizae Radix et Rhizoma, and
5 g of red tea.

In some embodiments, the traditional Chinese medicinal materials used in the pharmaceutical composition are processed traditional Chinese medicinal materials, and for example, the Arctii Fructus is stir-fried Arctii Fructus.
In some embodiments, the traditional Chinese medicinal materials used in the pharmaceutical composition are pulverized; preferably, the pulverized medicinal powder passes through Sieve No. 1; more preferably, the pulverized powder passes through the Sieve No. 1 but not pass through Sieve No. 5.
In some embodiments, the water content of the pharmaceutical composition is less than 12%; preferably, less than or equal to 10%, or less than or equal to 8%; more preferably, less than or equal to 6%, or less than or equal to 5%. The invention also provides a traditional Chinese medicine formulation for preventing and/or treating respiratory diseases in winter, comprising, by weight parts, the following traditional Chinese medicinal materials or extracts thereof in medicinal forms and pharmaceutically acceptable adjuvants:

2-4 parts by weight of Arctii Fructus,
1-3 parts by weight of Belamcandae Rhizoma,
1-3 parts by weight of Platycodonis Radi,
1-3 parts by weight of Paeoniae Radix Rubra,
1-3 parts by weight of Perillae folium,
2-4 parts by weight of Lonicerae Japonicae Flos,
1-3 parts by weight of Charred Hawthorn,
2-4 parts by weight of Astragali Radix,
2-4 parts by weight of Polygoni Cuspidati Rhizoma et Radix, and
0.5-1.5 parts by weight of Glycyrrhizae Radix et Rhizoma; as well as
0.5-8 parts by weight of non-essential fermented tea.

In some embodiments, the traditional Chinese medicine formulation comprises, by weight parts, the following traditional Chinese medicinal materials or extracts thereof in medicinal forms and pharmaceutically acceptable adjuvants:

2.5-3.5 parts by weight of Arctii Fructus,
1.5-2.5 parts by weight of Belamcandae Rhizoma,
1.5-2.5 parts by weight of Platycodonis Radi,
1.5-2.5 parts by weight of Paeoniae Radix Rubra,
1.5-2.5 parts by weight of Perillae folium,
2.5-3.5 parts by weight of Lonicerae Japonicae Flos,
1.5-2.5 parts by weight of Charred Hawthorn,
2.5-3.5 parts by weight of Astragali Radix,
2.5-3.5 parts by weight of Polygoni Cuspidati Rhizoma et Radix, and
0.8-1.2 parts by weight of Glycyrrhizae Radix et Rhizoma; as well as
2-6 parts by weight, preferably 3-5 parts by weight, of non-essential fermented tea, e.g., red tea, black tea or oolong tea.

In some embodiments, the traditional Chinese medicine formulation comprises, by weight parts, the following traditional Chinese medicinal materials or extracts thereof in medicinal forms and pharmaceutically acceptable adjuvants:

3 parts by weight of Arctii Fructus,
2 parts by weight of Belamcandae Rhizoma,
2 parts by weight of Platycodonis Radi,
2 parts by weight of Paeoniae Radix Rubra,
2 parts by weight of Perillae folium,
3 parts by weight of Lonicerae Japonicae Flos,
2 parts by weight of Charred Hawthorn,
3 parts by weight of Astragali Radix,
3 parts by weight of Polygoni Cuspidati Rhizoma et Radix, and
1 part by weight of Glycyrrhizae Radix et Rhizoma.

In some embodiments, the traditional Chinese medicine formulation comprises, by weight parts, the following traditional Chinese medicinal materials or extracts thereof in medicinal forms and pharmaceutically acceptable adjuvants:

3 parts by weight of Arctii Fructus,
2 parts by weight of Belamcandae Rhizoma,
2 parts by weight of Platycodonis Radi,
2 parts by weight of Paeoniae Radix Rubra,
2 parts by weight of Perillae folium,
3 parts by weight of Lonicerae Japonicae Flos,
2 parts by weight of Charred Hawthorn,
3 parts by weight of Astragali Radix,
3 parts by weight of Polygoni Cuspidati Rhizoma et Radix,
1 part by weight of Glycyrrhizae Radix et Rhizoma; and
3 parts by weight of red tea.

In some embodiments, the traditional Chinese medicine formulation comprises, by weight parts, the following traditional Chinese medicinal materials or extracts thereof in medicinal forms and pharmaceutically acceptable adjuvants:

3 parts by weight of Arctii Fructus,
2 parts by weight of Belamcandae Rhizoma,
2 parts by weight of Platycodonis Radi,
2 parts by weight of Paeoniae Radix Rubra,
2 parts by weight of Perillae folium,
3 parts by weight of Lonicerae Japonicae Flos,
2 parts by weight of Charred Hawthorn,
3 parts by weight of Astragali Radix,
3 parts by weight of Polygoni Cuspidati Rhizoma et Radix,
1 part by weight of Glycyrrhizae Radix et Rhizoma; and
4 parts by weight of red tea.

In some embodiments, the traditional Chinese medicine formulation comprises, by weight parts, the following traditional Chinese medicinal materials or extracts thereof in medicinal forms and pharmaceutically acceptable adjuvants:

3 parts by weight of Arctii Fructus,
2 parts by weight of Belamcandae Rhizoma,
2 parts by weight of Platycodonis Radi,
2 parts by weight of Paeoniae Radix Rubra,
2 parts by weight of Perillae folium,
3 parts by weight of Lonicerae Japonicae Flos,
2 parts by weight of Charred Hawthorn,
3 parts by weight of Astragali Radix,
3 parts by weight of Polygoni Cuspidati Rhizoma et Radix,
1 part by weight of Glycyrrhizae Radix et Rhizoma; and
5 parts by weight of red tea.

In preferred embodiments, the pharmaceutical composition is in a single dose form comprising the following traditional Chinese medicinal materials or extracts thereof in medicinal forms by weight parts:

3 g of stir-dried Arctii Fructus,
2 g of Belamcandae Rhizoma,
2 g of Platycodonis Radi,
2 g of Paeoniae Radix Rubra,
2 g of Perillae folium,
3 g of Lonicerae Japonicae Flos,
2 g of Charred Hawthorn,
3 g of Astragali Radix,
3 g of Polygoni Cuspidati Rhizoma et Radix, and
1 g of Glycyrrhizae Radix et Rhizoma.

In preferred embodiments, the pharmaceutical composition is in a single dose form comprising the following traditional Chinese medicinal materials or extracts thereof in medicinal forms by weight parts:

3 g of stir-dried Arctii Fructus,
2 g of Belamcandae Rhizoma,
2 g of Platycodonis Radi,
2 g of Paeoniae Radix Rubra,
2 g of Perillae folium,
3 g of Lonicerae Japonicae Flos,
2 g of Charred Hawthorn,
3 g of Astragali Radix,
3 g of Polygoni Cuspidati Rhizoma et Radix,
1 g of Glycyrrhizae Radix et Rhizoma, and
3 g of red tea.

In preferred embodiments, the pharmaceutical composition is in a single dose form comprising the following traditional Chinese medicinal materials or extracts thereof in medicinal forms by weight parts:

3 g of stir-dried Arctii Fructus,
2 g of Belamcandae Rhizoma,
2 g of Platycodonis Radi,
2 g of Paeoniae Radix Rubra,
2 g of Perillae folium,
3 g of Lonicerae Japonicae Flos,
2 g of Charred Hawthorn,
3 g of Astragali Radix,
3 g of Polygoni Cuspidati Rhizoma et Radix,
1 g of Glycyrrhizae Radix et Rhizoma, and
4 g of red tea.

In preferred embodiments, the pharmaceutical composition is in a single dose form comprising the following traditional Chinese medicinal materials or extracts thereof in medicinal forms by weight parts:

3 g of stir-dried Arctii Fructus,
2 g of Belamcandae Rhizoma,
2 g of Platycodonis Radi,
2 g of Paeoniae Radix Rubra,
2 g of Perillae folium,
3 g of Lonicerae Japonicae Flos,
2 g of Charred Hawthorn,
3 g of Astragali Radix,
3 g of Polygoni Cuspidati Rhizoma et Radix,
1 g of Glycyrrhizae Radix et Rhizoma, and
5 g of red tea.

In some embodiments, the pharmaceutical composition is formulated in a single dose form, comprising 2-10 g, preferably 3-6 g, 3-5 g, 3-4.5 g, 4-6 g, or 4-5 g, more preferably 3 g or 4.5 g of the pharmaceutical composition of any of the above embodiments.

In some embodiments, by per gram of the pharmaceutical composition, in the traditional Chinese medicine formulation:

the content of arctiin is $\geq$ 4.4 mg;
preferably, the content of arctiin is $\geq$5.5 mg;
more preferably, the content of arctiin is $\geq$7 mg or $\geq$7.3 mg.

In some embodiments, by 4.5 grams of the pharmaceutical composition, in the traditional Chinese medicine formulation:

the content of arctiin is $\geq$19.8 mg;
preferably, the content of arctiin is $\geq$20 mg.

In some embodiments, the content of arctiin is detected by high performance liquid chromatography, and the chromatographic conditions are shown below: Chromatographic column: Syncronis-AQ C18 (e.g., 250 mm$\times$4.6 mm, 5 $\mu$m); Mobile phase: acetonitrile (A) and water (B);

Flow rate: 0.8-1.2 mL$\cdot$min$^{-1}$;
Wavelength: 230 nm;
Sampling quantity: 5-10 $\mu$L;
Column temperature: 30 °C (20-35 °C);
Gradient elution procedure: 0-10 min, 14%-18% A; 10-18 min, 18% A; 18-22 min, 18%-9% A; 22-42 min, 19%-31% A; 42-50 min, 31%-55% A.

The invention also provides a traditional Chinese medicinal oral liquid for preventing and/or treating respiratory diseases in winter, comprising, by weight parts, extraction solutions, preferably water extraction solutions, of the following traditional Chinese medicinal decoction pieces, and pharmaceutically acceptable adjuvants:

2-4 parts by weight of Arctii Fructus,
1-3 parts by weight of Belamcandae Rhizoma,
1-3 parts by weight of Platycodonis Radi,
1-3 parts by weight of Paeoniae Radix Rubra,
1-3 parts by weight of Perillae folium,
2-4 parts by weight of Lonicerae Japonicae Flos,
1-3 parts by weight of Charred Hawthorn,
2-4 parts by weight of Astragali Radix,
2-4 parts by weight of Polygoni Cuspidati Rhizoma et Radix, and
0.5-1.5 parts by weight of Glycyrrhizae Radix et Rhizoma; as well as
0.5-8 parts by weight of non-essential fermented tea.

In some embodiments, the traditional Chinese medicinal oral liquid comprises, by weight parts, extraction solutions, preferably water extraction solutions, of the following traditional Chinese medicinal decoction pieces, and pharmaceutically acceptable adjuvants:

2.5-3.5 parts by weight of Arctii Fructus,
1.5-2.5 parts by weight of Belamcandae Rhizoma,
1.5-2.5 parts by weight of Platycodonis Radi,
1.5-2.5 parts by weight of Paeoniae Radix Rubra,
1.5-2.5 parts by weight of Perillae folium,
2.5-3.5 parts by weight of Lonicerae Japonicae Flos,
1.5-2.5 parts by weight of Charred Hawthorn,
2.5-3.5 parts by weight of Astragali Radix,
2.5-3.5 parts by weight of Polygoni Cuspidati Rhizoma et Radix, and
0.8-1.2 parts by weight of Glycyrrhizae Radix et Rhizoma; as well as
2-6 parts by weight, preferably 3-5 parts by weight, of non-essential fermented tea, e.g., red tea, black tea or oolong tea.

In some embodiments, the traditional Chinese medicinal oral liquid comprises, by weight parts, extraction solutions, preferably water extraction solutions, of the following traditional Chinese medicinal decoction pieces, and pharmaceutically acceptable adjuvants:

3 parts by weight of Arctii Fructus,
2 parts by weight of Belamcandae Rhizoma,
2 parts by weight of Platycodonis Radi,
2 parts by weight of Paeoniae Radix Rubra,
2 parts by weight of Perillae folium,
3 parts by weight of Lonicerae Japonicae Flos,
2 parts by weight of Charred Hawthorn,
3 parts by weight of Astragali Radix,
2 parts by weight of Polygoni Cuspidati Rhizoma et Radix, and
1 part by weight of Glycyrrhizae Radix et Rhizoma.

In some embodiments, the traditional Chinese medicinal oral liquid comprises, by weight parts, extraction solutions, preferably water extraction solutions, of the following traditional Chinese medicinal decoction pieces, and pharmaceutically acceptable adjuvants:

3 parts by weight of Arctii Fructus,
2 parts by weight of Belamcandae Rhizoma,
2 parts by weight of Platycodonis Radi,
2 parts by weight of Paeoniae Radix Rubra,
2 parts by weight of Perillae folium,
3 parts by weight of Lonicerae Japonicae Flos,
2 parts by weight of Charred Hawthorn,

3 parts by weight of Astragali Radix,
3 parts by weight of Polygoni Cuspidati Rhizoma et Radix,
1 part by weight of Glycyrrhizae Radix et Rhizoma; and
3 parts by weight of red tea.

In some embodiments, the traditional Chinese medicinal oral liquid comprises, by weight parts, extraction solutions, preferably water extraction solutions, of the following traditional Chinese medicinal decoction pieces, and pharmaceutically acceptable adjuvants:

3 parts by weight of Arctii Fructus,
2 parts by weight of Belamcandae Rhizoma,
2 parts by weight of Platycodonis Radi,
2 parts by weight of Paeoniae Radix Rubra,
2 parts by weight of Perillae folium,
3 parts by weight of Lonicerae Japonicae Flos,
2 parts by weight of Charred Hawthorn,
3 parts by weight of Astragali Radix,
3 parts by weight of Polygoni Cuspidati Rhizoma et Radix,
1 part by weight of Glycyrrhizae Radix et Rhizoma; and
4 parts by weight of red tea.

In some embodiments, the traditional Chinese medicinal oral liquid comprises, by weight parts, extraction solutions, preferably water extraction solutions, of the following traditional Chinese medicinal decoction pieces, and pharmaceutically acceptable adjuvants:

3 parts by weight of Arctii Fructus,
2 parts by weight of Belamcandae Rhizoma,
2 parts by weight of Platycodonis Radi,
2 parts by weight of Paeoniae Radix Rubra,
2 parts by weight of Perillae folium,
3 parts by weight of Lonicerae Japonicae Flos,
2 parts by weight of Charred Hawthorn,
3 parts by weight of Astragali Radix,
3 parts by weight of Polygoni Cuspidati Rhizoma et Radix,
1 part by weight of Glycyrrhizae Radix et Rhizoma; and
5 parts by weight of red tea.

In preferred embodiments, the traditional Chinese medicinal oral liquid is in a single dose form comprising, by weight parts, extraction solutions, preferably water extraction solutions, of the following traditional Chinese medicinal decoction pieces, and pharmaceutically acceptable adjuvants:

3 g of stir-dried Arctii Fructus,
2 g of Belamcandae Rhizoma,
2 g of Platycodonis Radi,
2 g of Paeoniae Radix Rubra,
2 g of Perillae folium,
3 g of Lonicerae Japonicae Flos,
2 g of Charred Hawthorn,
3 g of Astragali Radix,
3 g of Polygoni Cuspidati Rhizoma et Radix, and
1 g of Glycyrrhizae Radix et Rhizoma.

In preferred embodiments, the traditional Chinese medicinal oral liquid is in a single dose form comprising, by weight parts, extraction solutions, preferably water extraction solutions, of the following traditional Chinese medicinal decoction pieces, and pharmaceutically acceptable adjuvants:

3 g of stir-dried Arctii Fructus,
2 g of Belamcandae Rhizoma,

2 g of Platycodonis Radi,
2 g of Paeoniae Radix Rubra,
2 g of Perillae folium,
3 g of Lonicerae Japonicae Flos,
2 g of Charred Hawthorn,
3 g of Astragali Radix,
3 g of Polygoni Cuspidati Rhizoma et Radix,
1 g of Glycyrrhizae Radix et Rhizoma, and
3 g of red tea.

In preferred embodiments, the traditional Chinese medicinal oral liquid is in a single dose form comprising, by weight parts, extraction solutions, preferably water extraction solutions, of the following traditional Chinese medicinal decoction pieces, and pharmaceutically acceptable adjuvants:

3 g of stir-dried Arctii Fructus,
2 g of Belamcandae Rhizoma,
2 g of Platycodonis Radi,
2 g of Paeoniae Radix Rubra,
2 g of Perillae folium,
3 g of Lonicerae Japonicae Flos,
2 g of Charred Hawthorn,
3 g of Astragali Radix,
3 g of Polygoni Cuspidati Rhizoma et Radix,
1 g of Glycyrrhizae Radix et Rhizoma, and
4 g of red tea.

In preferred embodiments, the traditional Chinese medicinal oral liquid is in a single dose form comprising, by weight parts, extraction solutions, preferably water extraction solutions, of the following traditional Chinese medicinal decoction pieces, and pharmaceutically acceptable adjuvants:

3 g of stir-dried Arctii Fructus,
2 g of Belamcandae Rhizoma,
2 g of Platycodonis Radi,
2 g of Paeoniae Radix Rubra,
2 g of Perillae folium,
3 g of Lonicerae Japonicae Flos,
2 g of Charred Hawthorn,
3 g of Astragali Radix,
3 g of Polygoni Cuspidati Rhizoma et Radix,
1 g of Glycyrrhizae Radix et Rhizoma, and
5 g of red tea.

The invention also provides a traditional Chinese medicinal tea bag formulation for preventing and/or treating respiratory diseases in winter, comprising decoction piece powders of the following traditional Chinese medicine materials and acceptable additives in food or beverage by weight parts:

2-4 parts by weight of Arctii Fructus,
1-3 parts by weight of Belamcandae Rhizoma,
1-3 parts by weight of Platycodonis Radi,
1-3 parts by weight of Paeoniae Radix Rubra,
1-3 parts by weight of Perillae folium,
2-4 parts by weight of Lonicerae Japonicae Flos,
1-3 parts by weight of Charred Hawthorn,
2-4 parts by weight of Astragali Radix,
2-4 parts by weight of Polygoni Cuspidati Rhizoma et Radix, and
0.5-1.5 parts by weight of Glycyrrhizae Radix et Rhizoma; as well as
0.5-8 parts by weight of non-essential fermented tea.

In some embodiments, the traditional Chinese medicine tea bag formulation comprises decoction piece powders of the following traditional Chinese medicine materials and acceptable additives in food or beverage by weight parts:

2.5-3.5 parts by weight of Arctii Fructus,
1.5-2.5 parts by weight of Belamcandae Rhizoma,
1.5-2.5 parts by weight of Platycodonis Radi,
1.5-2.5 parts by weight of Paeoniae Radix Rubra,
1.5-2.5 parts by weight of Perillae folium,
2.5-3.5 parts by weight of Lonicerae Japonicae Flos,
1.5-2.5 parts by weight of Charred Hawthorn,
2.5-3.5 parts by weight of Astragali Radix,
2.5-3.5 parts by weight of Polygoni Cuspidati Rhizoma et Radix, and
0.8-1.2 parts by weight of Glycyrrhizae Radix et Rhizoma; as well as
2-6 parts by weight, preferably 3-5 parts by weight, of non-essential fermented tea, e.g., red tea, black tea or oolong tea.

In some embodiments, the traditional Chinese medicine tea bag formulation comprises decoction piece powders of the following traditional Chinese medicine materials and acceptable additives in food or beverage by weight parts:

3 parts by weight of Arctii Fructus,
2 parts by weight of Belamcandae Rhizoma,
2 parts by weight of Platycodonis Radi,
2 parts by weight of Paeoniae Radix Rubra,
2 parts by weight of Perillae folium,
3 parts by weight of Lonicerae Japonicae Flos,
2 parts by weight of Charred Hawthorn,
3 parts by weight of Astragali Radix,
3 parts by weight of Polygoni Cuspidati Rhizoma et Radix, and
1 part by weight of Glycyrrhizae Radix et Rhizoma.

In some embodiments, the traditional Chinese medicine tea bag formulation comprises decoction piece powders of the following traditional Chinese medicine materials and acceptable additives in food or beverage by weight parts:

3 parts by weight of Arctii Fructus,
2 parts by weight of Belamcandae Rhizoma,
2 parts by weight of Platycodonis Radi,
2 parts by weight of Paeoniae Radix Rubra,
2 parts by weight of Perillae folium,
3 parts by weight of Lonicerae Japonicae Flos,
2 parts by weight of Charred Hawthorn,
3 parts by weight of Astragali Radix,
3 parts by weight of Polygoni Cuspidati Rhizoma et Radix,
1 part by weight of Glycyrrhizae Radix et Rhizoma; and
3 parts by weight of red tea.

In some embodiments, the traditional Chinese medicine tea bag formulation comprises decoction piece powders of the following traditional Chinese medicine materials and acceptable additives in food or beverage by weight parts:

3 parts by weight of Arctii Fructus,
2 parts by weight of Belamcandae Rhizoma,
2 parts by weight of Platycodonis Radi,
2 parts by weight of Paeoniae Radix Rubra,
2 parts by weight of Perillae folium,
3 parts by weight of Lonicerae Japonicae Flos,
2 parts by weight of Charred Hawthorn,
3 parts by weight of Astragali Radix,
3 parts by weight of Polygoni Cuspidati Rhizoma et Radix,
1 part by weight of Glycyrrhizae Radix et Rhizoma; and

4 parts by weight of red tea.

In some embodiments, the traditional Chinese medicine tea bag formulation comprises decoction piece powders of the following traditional Chinese medicine materials and acceptable additives in food or beverage by weight parts:

3 parts by weight of Arctii Fructus,
2 parts by weight of Belamcandae Rhizoma,
2 parts by weight of Platycodonis Radi,
2 parts by weight of Paeoniae Radix Rubra,
2 parts by weight of Perillae folium,
3 parts by weight of Lonicerae Japonicae Flos,
2 parts by weight of Charred Hawthorn,
3 parts by weight of Astragali Radix,
3 parts by weight of Polygoni Cuspidati Rhizoma et Radix,
1 part by weight of Glycyrrhizae Radix et Rhizoma; and
5 parts by weight of red tea.

As appreciated by those skilled in the art, the weight parts of the above traditional Chinese medicinal materials are relative, and based on traditional Chinese medicinal theories, the amounts of one or more traditional Chinese medicinal materials may be reasonably adjusted according to actual needs. All evident variable forms of prescriptions comprising component with such reasonably adjusted amounts are within the scope of the invention.

As above described, the traditional Chinese medicine formulation according to the invention, in addition to the traditional Chinese medicine composition of the invention, may further comprise pharmaceutically acceptable adjuvants. Pharmaceutically acceptable adjuvants that can be used in the traditional Chinese medicine composition of the invention include any adjuvants commonly used in traditional Chinese medicine formulations, as long as the adjuvants do not adversely affect the quality, performances and therapeutic effects of the traditional Chinese medicine composition of the invention. Adjuvants commonly used in traditional Chinese medicine formulations include diluents, carriers, fillers, binders, wetting agents, disintegrating agents, absorption promoters, surfactants, adsorption carriers and lubricants. Commonly-used diluents mainly include sucrose, dextrin, starch, lactose, mannitol, xylitol, bifidus sugar, and the like. Commonly-used wetting agents mainly comprise water, ethanol with different concentrations and the like; commonly-used binders include polymer binders in a wide variety such as ethylcellulose, polyvinylpyrrolidone, sodium carboxymethylcellulose, polyethylene glycol, sodium alginate, and the like. Commonly-used disintegrating agents include microcrystalline cellulose, sodium carboxymethyl starch and the like. Those skilled in the art can select and determine suitable adjuvants in the traditional Chinese medicine formulation of the invention according to the disclosures in the specification. The choice of specific adjuvants will depend on administration modes for treating specific patents or types and states of diseases.

If necessary, the traditional Chinese medicine formulation of the invention may further comprise suitable additives. These additives are known in the art, for example, emulsifiers, fragrances, solubilizers, anticaking agents, antifoaming agents, binders, buffering agents, pH adjusters, propellants, chelating agents, preservatives and the like.

The traditional Chinese medicine composition and traditional Chinese medicine formulation of the invention can be easily prepared by those skilled in the art according to the methods described in the specification and known techniques in the art.

For example, those skilled in the art can prepare the pharmaceutical composition of the invention by a method comprising the following steps:

(1) pulverizing individual medicinal materials respectively, and passing them through Sieve No. 1 and Sieve No. 5 to obtain a coarse powder between Sieve No. 1 and Sieve No. 5; preferably, further pulverizing the powder passing on Sieve No. 1 and then passing it through Sieve No. 1 and Sieve No. 5, to obtain a coarse powder between Sieve No. 1 and Sieve No. 5 which is combined with the previously obtained coarse powder;
(2) uniformly mixing the obtained coarse powders;
(3) packaging and sterilizing;

preferably, in the step (1), Arctii Fructus and Paeoniae Radix Rubra are pulverized together; preferably, the other medicinal materials are pulverized separately or together.

On this basis, those skilled in the art can easily prepare the traditional Chinese medicine formulation of the invention in a desired dosage according to the disclosure of the specification and known techniques in the art.

For the steps of extracting, filtering, concentrating, drying and sub-packaging involved in the process for preparation of the traditional Chinese medicine formulation of the invention, those skilled in the art can implement them with commonly-used methods and apparatus in the art, e.g., to filter an extraction solution or a filtrate with, e.g., 100- to 300-mesh sieves.

As appreciated by those skilled in the art, based on the disclosure of the specification, corresponding traditional Chinese medicinal materials may be used alone or in a mixture thereof, to prepare traditional Chinese medicinal active extracts and traditional Chinese medicine formulations applicable in the invention by conventional pulverizing, immersion extracting, and separating methods in the art, such as immersion, percolation, liquid-liquid extraction, water-extraction and alcohol precipitation, alcohol extraction and water precipitation, and dialysis. One or several active extracts in the traditional Chinese medicines used in the invention may be purchased in commercial routes and then formulated with the extracts of the other traditional Chinese medicines, to obtain the traditional Chinese medicinal active extracts of the invention. Traditional Chinese medicine compositions and traditional Chinese medicine formulations in these variable forms are in the scope of the invention.

The traditional Chinese medicine composition and traditional Chinese medicine formulation of the invention may be in a unit dose form. The term "unit dose form" means a physically dispersed unit suitable as a unit dose that may be administrated on human subjects and other mammals, each unit comprising the pharmaceutical composition and pharmaceutical formulations of the invention in predetermined amounts that, by making calculations, can produce desired therapeutic effects, and suitable pharmaceutically acceptable adjuvants.

The traditional Chinese medicine composition and traditional Chinese medicine formulation of the invention may significantly inhibit the release of cytokines TNF-$\alpha$, IL-6 and IL-1$\beta$ of LPS-induced THP-1 cells, indicating that they have anti-inflammatory effects. The results obtained with acute pharyngitis rat models show that the pharmaceutical composition and traditional Chinese medicine formulation of the invention have evident effects of preventing and treating acute pharyngitis caused by pathogenic microorganisms, e.g., Staphylococcus aureus.

Therefore, the traditional Chinese medicine composition and the traditional Chinese medicine formulation of the invention may be applied in uses as described in one, more or all of the following items 1) to 5):

1) nourishing Qi, moistening lungs, clearing throats, relieving sore throats, and/or decontaminating,
2) treating and preventing seasonal respiratory diseases which is preferably influenza;
3) treating and preventing pharyngitis which is preferably acute pharyngitis;
4) eliminating phlegm; and
5) relieving cough;

comprising administrating effective amounts of the traditional Chinese medicine composition and traditional Chinese medicine formulation of the invention to a subject in need, e.g., as tea bag, 1 bag per day (3g/bag or 4.5 g/bag), 2-3 times per day.

Alternatively, the traditional Chinese medicine composition of the invention may be used for the manufacture of medicaments as described in one, more or all of the following items 1) to 4):

1) medicaments for nourishing Qi, moistening lungs, clearing throats, relieving sore throats, and/or decontaminating,
2) medicaments for treating and preventing pharyngitis which is preferably acute pharyngitis;
3) medicaments for eliminating phlegm; and
4) medicaments relieving cough.

The traditional Chinese medicine composition and traditional Chinese medicine formulation of the invention may be administrated in any suitable modes and any suitable forms that are commonly used in the art. Preferably, the traditional Chinese medicine composition and traditional Chinese medicine formulation of the invention may be orally administration. For example, the traditional Chinese medicine composition and traditional Chinese medicine formulation of the invention may be orally taken twice in one day, for each time, 5 to 30 g, for example 10 to 20 g, preferably 10 g of the traditional Chinese medicine composition and traditional Chinese medicine formulation of the invention. Specific administration doses depend on factors such as weights of subjects, properties and serenity of diseases, administration modes of medicaments and administration cycles or time interval. As for some patients in special situations, particular administration modes should follow doctor's advices.

The traditional Chinese medicine composition and traditional Chinese medicine formulation of the invention may be used in combination with other medicaments and techniques for preventing and treating respiratory diseases that are known in the art.

The traditional Chinese medicine composition and traditional Chinese medicine formulation are expected to produce the technical effects as described in in one, more or all of the following items (1) to (5):

(1) nourishing yin and relieving sore throat;
(2) nourishing Qi, moistening lungs, clearing throats, relieving sore throats, and decontaminating,

(3) effectively treating and preventing pharyngitis;
(4) effectively eliminating phlegm; and
(5) effectively relieving cough.

**Examples**

Embodiments of the invention will be described in detail below by combining examples. However, it will be appreciated by those skilled in the art that the following examples are only illustrative for the invention and they should not be contemplated to limit the scope of the invention. In the examples, those that are not specified in terms of specific conditions are conducted according to conventional conditions or conditions recommended by manufacturers. The used reagents or apparatus that are not specified in terms of manufacturers each are conventional commercially available products.

Methods for formulating medicinal solutions of the inventive pharmaceutical compositions used in the preparation examples of the invention each are to formulate a medicinal solution at a desired concentration with distillated water at room temperature, which is fully mixed with ultrasonic treatments for 20 minutes, not in need of removing dregs of a decoction, and to take the supernatant for administration, further dilution or storage for use.

**Preparation Example 1: Preparation of the pharmaceutical compositions and formulations of the invention**

Prescription:

3 g of stir-dried Arctii Fructus,
2 g of Belamcandae Rhizoma,
2 g of Platycodonis Radi,
2 g of Paeoniae Radix Rubra,
2 g of Perillae folium,
3 g of Lonicerae Japonicae Flos,
2 g of Charred Hawthorn,
3 g of Astragali Radix,
3 g of Polygoni Cuspidati Rhizoma et Radix,
1 g of Glycyrrhizae Radix et Rhizoma, and
4 g of red tea.

The above individual medicinal materials were pulverized separately and all sieved by passing through Sieve No. 1 (10 mesh) to form coarse powders between Sieve No. 1 (10 mesh) and Sieve No. 5 (80 meshes), which, by charging them in a quantity of 1000 prescriptions according to the formulation proportions in the above prescriptions, were weighed in a total quantity of 27.0 kg, and the resultant powders were charged in a mixing machine for total mixing (the total mixing is aimed to enabling all the powders of medicinal materials to be uniformly mixed) for 60 minutes, to obtain a medicinal powder in a quantity of 26.9 kg, which, after sterilization, was packaged with tea filter paper of heat-sealing type into 3 g/bag or 4.5 g/bag (the yield in inner packaging process: 98-99.5%), and then was externally packaged to obtain a finished product (batch number: SJD2020001).

**Preparation Example 2: Preparation of the pharmaceutical compositions and formulations of the invention**

The prescription is the same as that in Preparation Example 1.

The above medicinal materials, by charging them in a quantity of 1000 prescriptions according to the formulation proportions in the above prescriptions, were weighed in a total quantity of 27.0 kg, and after the decoction pieces were mixed, the mixture was pulverized so that it totally passed through Sieve No. 1 (10 mesh) to form a coarse powder between Sieve No. 1 (10 mesh) and Sieve No. 5 (80 meshes). The resultant powder was charged in a mixing machine for total mixing (the total mixing is aimed to enabling all the powders of medicinal materials to be uniformly mixed) for 30 minutes, to obtain a medicinal powder in a quantity of 26.8 kg, which, after sterilization, was packaged with tea filter paper of heat-sealing type into 3 g/bag or 4.5 g/bag (the yield in inner packaging process: 98-99.6%), and then was externally packaged to obtain a finished product (batch number: SJD2020001).

**Preparation Example 3: Preparation of the pharmaceutical compositions and formulations of the invention**

The prescription is the same as that in Preparation Example 1.

The above medicinal materials, by charging them in a quantity of 1000 prescriptions according to the formulation proportions in the above prescriptions, were weighed in a total quantity of 27.0 kg, and after the decoction pieces were mixed, the mixture was pulverized so that it totally passed through Sieve No. 1 to form a coarse powder between Sieve No. 1 and Sieve No. 5 (80 meshes). The resultant powder was charged in a mixing machine for total mixing (the total mixing is aimed to enabling all the powders of medicinal materials to be uniformly mixed) for 30 minutes, to obtain a medicinal powder in a quantity of 26.7 kg, which, after sterilization, was packaged with tea filter paper of heat-sealing type into 3 g/bag or 4.5 g/bag (the yield in inner packaging process: 99.7%), and then was externally packaged to obtain a finished product (batch number: SJD2020002).

**Experimental Example 1: Quality detections and controls**

Presently, Thin-Layer Chromatography (TLC) was utilized to conduct qualitative identifications to one main medicament in the sample, Paeoniae Radix Rubra, and the other medicament, Belamcandae Rhizoma, and High Performance Liquid Chromatography (HPLC) was utilized to measure the content of one main medicament, Arctii Fructus. According to the standards of tea formulations (Pharmacopoeia of the People's Republic of China (2015 version), Part IV, Tea Formulations, General Rules 0188), an overall study on quality standards was conducted to provide scientific and effective bases for further studying the formulations.

**1 Experimental Materials and Methods**

**1.1 Experimental Materials and Reagents**

**1.1.1 Instruments**

| Name | Manufactures |
| --- | --- |
| Thermo Ultimate3000-type High Performance Liquid Chromatograph | Thermo Fisher Scientific |
| Syncronis-AQ C18 chromatographic column (250 mm × 4.6 mm, 5 μm) | Thermo Fisher Scientific |
| SQP QUINTIX224-1CN Sartorius scale | Sartorius AG, German |
| ME403T/02 electronic scale | Mettler Toled (Shanghai) |
| AS20500BT ultrasonic cleaner | Tianjing Otter Saines |

**1.1.2 Reagents and Materials**

| Names | Manufactures |
| --- | --- |
| Methanol, ethanol, ethyl acetate | Tianjin Northern Tianyi Chemical Reaqent Factory |
| Ultra-pure water | Cascada™ Ultra-pure Water Machine from Pall Corporation, USA |
| Formic acid (chromatogram pure) | Tianjing Guangfu Fine Chemical Research Institute |
| Trichloromethane, cyclohexane, petroleum ether | Tianjin Jiangtian Chemical Technique Ltd. |
| Silica gel G board | Branch Factory of Qingdao Marine Chemistry Factory |
| 0.3 mm glass sampling capillary | West China Medical University Instrument Factory |

**1.1.3 Control Samples**

Control samples for content measurement: Arctiin (batch No.: 110819-201812, purity: 95.0%), commercially available from National Institutes for Food and Drug Control.

Control samples for thin-layer identification: Peoniflorin (batch No.: 110736-201943, putridity: 95.1%), Irisflorentin (batch No.: 11557-201703, purity: 99.9%) and Belamcandae Rhizoma control medicinal material (batch No.: 120994-201206), each commercially available from National Institutes for Food and Drug Control.

**1.1.4 Samples**

According to Preparation Examples 1-3, the pharmaceutical compositions (SJD2020001, SJD202001, SJD2020002, SJD2020003 and SJD2020004) of the invention were prepared, and meanwhile, according to the same method, batches of samples lack of Paeoniae Radix Rubra, Belamcandae Rhizoma and Polygoni Cuspidati Rhizoma et Radix were respectively prepared as negative controls.

In negative control 1, except for the lack of Paeoniae Radix Rubra, the other medicinal materials and amounts thereof, and preparation processes each are the same as those in Preparation Example 3. A negative sample was provided in order to prove that the method for identification of Paeoniae Radix Rubra in samples is reliable, and to show that the characteristic spot of the Peoniflorin in Paeoniae Radix Rubra is really present in the samples, and in addition to Paeoniae Radix Rubra, the other medicinal materials are free of the Peoniflorin.

In negative control 2, except for the lack of Belamcandae Rhizoma, the other medicinal materials and amounts thereof, and preparation processes each are the same as those in Preparation Example 3. A negative sample was provided in order to prove that the method for identification of Belamcandae Rhizoma in samples is reliable, and to show that the characteristic spots of an Irisflorentin control sample (111557-201703) in the Belamcandae Rhizoma, and in addition to Belamcandae Rhizoma, the other medicinal materials are free of the Irisflorentin.

In negative control 3, except for the lack of Polygoni Cuspidati Rhizoma et Radix, the other medicinal materials and amounts thereof, and preparation processes each are the same as those in Preparation Example 3. A negative sample was provided in order to prove that the method for identification of Polygoni Cuspidati Rhizoma et Radix in samples is reliable, and to show the characteristic spot of a Polydatin control sample in Polygoni Cuspidati Rhizoma et Radix is really present in the sample, and in addition to Polygoni Cuspidati Rhizoma et Radix, the other medicinal materials are free of the Polydatin.

**1.2 Experimental Methods**

**1.2.1 Thin-Layer Identification**

**1.2.1.1 Thin-Layer Identification of Paeoniae Radix Rubra**

(1) Preparations of solutions of test samples and negative test samples

2 g of a medicinal powder of the sample (prepared in Preparation Example 3) were taken and added with 40 mL of ethanol, and they were shaken and filtered. The filtrate was evaporated to dryness, and the residue was dissolved by adding 5 ml of ethanol as the test sample solution.

Negative control 1 was taken and prepared into a negative control solution according to the same method.

(2) Preparation of control medicinal material solutions

0.5 g of Paeoniae Radix Rubra control medicinal material were taken and prepared into a control medicinal material solution according to the same method as that described in item 1.2.1.1 (1).

(3) Formulation of control sample solutions

2 mg of a Peoniflorin control sample were taken and added with ethanol to formulate a control sample solution (2 mg·mL$^{-1}$).

(4) Developing conditions of thin-layer chromatography

Thin layer plate: silica gel G thin layer plate
Sample quantity: 5 $\mu$L of the above individual solutions
Developing agent: trifluoromethane-ethyl acetate-methanol-formic acid (40:5:10:0.2) Chromogenic agent: 5% vanillin-sulfuric acid solution, developing color by heating at 105 °C

**1.2.1.2 Thin-Layer Identification of Belamcandae Rhizoma**

(1) Preparations of solutions of test samples and negative test samples

2 g of medicinal powders of the samples (prepared in Preparation Examples 1-3) were taken and added with 40 mL of ethanol, and they were shaken and filtered. The filtrate was evaporated to dryness, and the residue was dissolved by adding 5 ml of ethanol as the test sample solution.

Negative control 2 was taken and prepared into a negative control solution according to the same method.

(2) Preparation of control medicinal material solutions

0.5 g of Belamcandae Rhizoma control medicinal material were taken and prepared into a control medicinal material solution according to the same method as that described in item 1.2.1.1 (1).

(3) Formulation of control sample solutions

2 mg of an Irisflorentin control sample were taken and added with ethanol to formulate a control sample solution (2 mg•mL$^{-1}$).

(4) Developing conditions of thin-layer chromatography

Thin layer plate: silica gel G thin layer plate
Sample quantity: 5 $\mu$L of the above individual solutions
Developing agent: petroleum ether (60-90 °C)-ethyl acetate (1:1)
Chromogenic agent: observed at 254 nm

**1.2.1.3 Thin-Layer Identification of Polygoni Cuspidati Rhizoma et Radix**

(1) Preparations of solutions of test samples and negative test samples

2 g of medicinal powders of the samples (prepared in Preparation Examples 1-3) were taken and added with 20 mL of ethanol, and they were ultrasonically treated from 20 minutes and filtered. The filtrate was evaporated to dryness, and the residue was dissolved by adding 4 ml of methanol as the test sample solutions.

Negative control 3 was taken and prepared into a negative control solution according to the same method.

(2) Preparation of control medicinal material solutions

0.5 g of P Belamcandae Rhizoma control medicinal material were taken and prepared into a control medicinal material solution according to the same method as that described in item 1.2.1.1 (1).

(3) Formulation of control sample solutions

A Polygoni Cuspidati Rhizoma et Radix control sample were taken and added with ethanol to formulate a control sample solution (1 mg•mL$^{-1}$).

(4) Developing conditions of thin-layer chromatography

Thin layer plate: silica gel G thin layer plate
Sample quantity: 4-8 $\mu$l of the test sample solution; 1-2 $\mu$l of the control sample solution
Developing agent: difluoromethane-methanol-diethylamine (10:3:0.1)
Chromogenic agent: viewing under a 300 nm UV light

### 1.2.2 Inspections

Inspections were conducted according to Pharmacopoeia of the People's Republic of China (2015 version), Part IV, Tea Formulations, General Rules 0188.

### 1.2.2.1 Moisture measurements

Measurements were conducted according to Pharmacopoeia of the People's Republic of China (2015 version), Part IV, moisture measurements (General Rule 0832).

### 1.2.2.2 Loading Differences

The samples prepared in Preparation Examples 1-3 were taken in one batch respectively, 10 bags for each batch, and by inspecting according to the method of "loading difference" prescribed under Pharmacopoeia of the People's Republic of China (2015 version), Part IV, Tea Formulations, General Rules 0188, the weight of the products was 3 g, and the loading difference should be ±12%.

### 1.2.2.3 Microbial Limits

The samples prepared in Preparation Examples 1-3 were taken in one batch respectively, 3 bags for each batch, and according to the method of "Microbial Limits" prescribed under Pharmacopoeia of the People's Republic of China (2015 version), Part IV, Tea Formulations, General Rules 0188, the inspections were conducted.

### 1.2.3 Content Measurements

The samples prepared in Preparation Examples 1-3 were taken in one batch respectively, each batch including 3 samples, and 3 g for each sample.

Preparation of samples: 3 g of a sample was taken and placed in a flask, and it was added with 180 mL of 85 °C purified water and immersed for 30 min. The sample was taken and added with methanol, and it was ultrasonically treated for 30 min. After cooling, it was transferred into a centrifugal tube wherein it was centrifuged for 15 min (5000 rpm). The supernatant was transferred into a 10 mL volumetric flask, and it was kept at a constant volume with 90% methanol and shaken. By filtering with a 0.45 $\mu$m microporous filtration membrane, the sample was obtained for use.

Chromatographic conditions: chromatographic column: Syncronis-AQ C18 (250 mm×4.6 mm, 5 $\mu$m); mobile phase: acetonitrile (A) and water (B); flow rate: 0.8 mL·min$^{-1}$; wavelength: 230 nm; sampling quantity: 10 $\mu$L; column temperature: 30 °C. Gradient elution procedure: 0-10 min, 14%-18% A; 10-18 min, 18% A; 18-22 min, 18%-19% A; 22-42 min, 19%-31% A; 42-50 min, 31 %-55% A. The theoretical plate number, calculated according Arctiin peaks, was not less than 5000.

### 2 Experimental Results

### 2.1 Thin-Layer Identification Results

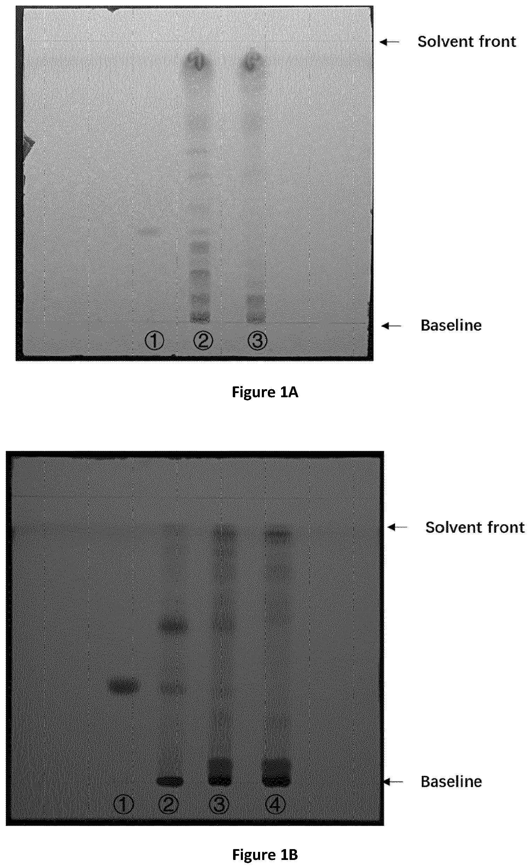

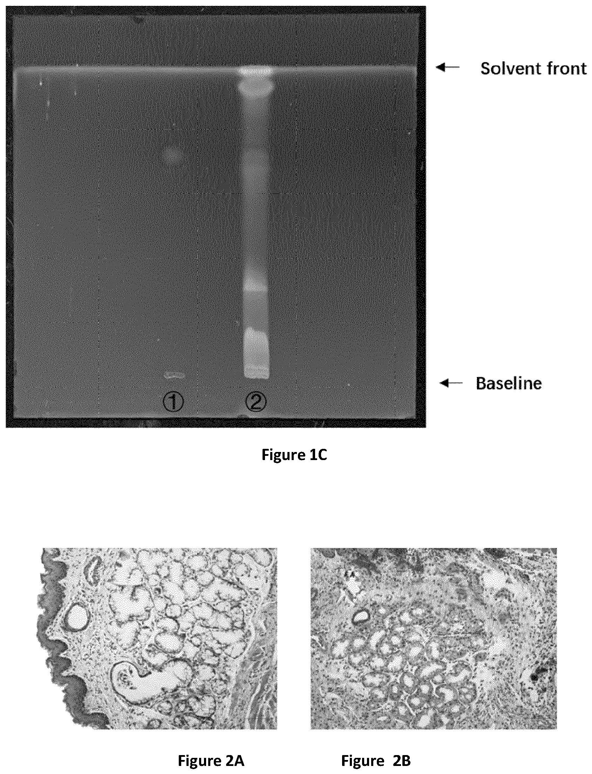

Thin-layer identification results were shown in Fig. 1A, Fig. 1B and Fig. 1C.

As can be seen from Fig. 1A, the inventive pharmaceutical composition samples each have the characteristic spot of the Peoniflorin, whilst in the negative samples lack of Paeoniae Radix Rubra, the characteristic spot of the Peoniflorin was not observed, to show that this method may be applied in the identification of Paeoniae Radix Rubra in the samples.

As can be seen from Fig. 1B, the inventive pharmaceutical composition samples each have the characteristic spot of the Irisflorentin, whilst in the negative samples lack of Belamcandae Rhizoma, the characteristic spot of the Irisflorentin was not observed, to show that this method may be applied in the identification of Belamcandae Rhizoma in the samples.

As can be seen from Fig. 1C, the inventive pharmaceutical composition samples each have the characteristic spot of the Polydatin, whilst in the samples lack of Polygoni Cuspidati Rhizoma et Radix, the characteristic spot of the Polydatin was not observed, to show that this method may be applied in the identification of Polygoni Cuspidati Rhizoma et Radix in the samples.

**2.2 Inspection Results of Tea Formulation General Rules**

**2.2.1 Inspection Results of Water Contents**

Results are shown in Table 1.

Table 1: Inspection Results of Water Contents

| Batch Number | Water contents (%) | Moisture Limits (%) |
|---|---|---|
| SJD2020002 | 6.0 | |
| SJD2020003 | 6.0 | <12 |
| SJD2020004 | 6.0 | |

As can be seen from Table 1, the water contents each were lower than 12% and in line with relevant requirements of tea formulations.

**2.2.2 Inspection Results of Loading Differences**

The results were shown in Table 2.

Table 2: Inspection Results of loading quantities

| Bag number/batch number | SJD2020002 | SJD2020003 | SJD2020004 |
|---|---|---|---|
| 1 | 4.302 | 4.622 | 4.433 |
| 2 | 4.428 | 4.422 | 4.473 |
| 3 | 4.228 | 4.553 | 4.651 |
| 4 | 4.425 | 4.443 | 4.444 |
| 5 | 4.263 | 4.602 | 4.163 |
| 6 | 4.562 | 4.557 | 4.321 |
| 7 | 4.478 | 4.449 | 4.479 |
| 8 | 4.347 | 4.580 | 4.457 |
| 9 | 4.513 | 4.385 | 4.255 |
| 10 | 4.332 | 4.578 | 4.322 |
| Average loading/g | 4.388 | 4.519 | 4.400 |
| Number of bags with granules exceeding limits | 0 | 0 | 0 |
| Number of bags with granules exceeding limits by one time | 0 | 0 | 0 |

As can be seen from Table 2, the loading differences each met the requirements of tea formulations.

**2.2.3 Inspection Results of Microorganisms**

Inspection results of the microorganisms in the three batches of samples met relevant provisions.

**2.3 Content Measurement Results**

An external standard method was used to calculate contents and take their average values, and the results were shown in Table 3.

Table 3: Content measurement results of three batches of samples (n=3)

| Batch number | Arctiin mg/g |
|---|---|
| SJD2020002 | 7.49 |
| SJD2020003 | 7.43 |
| SJD2020004 | 7.00 |
| Average value | 7.31 |

To more representatively determine the content limits of the index ingredients of the inventive pharmaceutical compositions, the inventor, based on the Arctiin content standards specified in Pharmacopoeia of the People's Republic of China, determined the content limits of the index ingredients of the inventive pharmaceutical compositions. The specific contents are shown below:

According to the provisions of Pharmacopoeia of the People's Republic of China (2015 version), the Arctiin in stir-fried Arctii Fructus decoction pieces is 5.0%, and according to preparation processes of tea formulations, the proportion of the Arctii Fructus in a prescription is 11.1%. By combining the average yield 99% of the inventive pharmaceutical compositions in the pilot magnification productions of three batches, the content was calculated to be 5.5 mg/g, by 20% downward floating, being 4.4 mg/g, and each bag (4.5 g) with 19.8 mg was used as the standard of the arctiin content in the inventive pharmaceutical compositions.

The content limit standard of the inventive pharmaceutical compositions: each bag of the inventive pharmaceutical compositions contained not less than 19.8 mg of Arctii Fructus, in Arctiin ($C_{27}H_{34}O_{11}$).

Experimental Example 2: Preventing and treating effects of the pharmaceutical compositions of the invention on rat models suffered from acute pharyngitis caused by Staphylococcus aureus

This experiment used rats suffered from acute pharyngitis caused by staphylococcus aureus, in the three aspects of appearance indexes, pathological indexes, and biochemical indexes, to evaluate the medicinal effects of the inventive pharmaceutical compositions, thereby providing scientific bases for clinical applications of the inventive pharmaceutical compositions.

**1. Materials and Methods**

**1.1 Experimental Instruments**

| Names | Manufacturers |
|---|---|
| VIDEO Visional Body Surface Detection Meter | Shanghai Wuzhou Electronic Technology Ltd |
| Weighing Scale for Experimental Animal | Shanghai Huachao Industry Ltd |
| Electronic Scale | Ohausyt (Shanghai) Instrument Ltd |
| Rat cage | Trade Firm of Fengqiao Danlo in New and High-tech Zones |
| Centrifuge | Beijing New Time Beili Medical Instrument Ltd |
| TECAN Enzyme-Labelled Meter | Beijing Haitianyoucheng Science and Technology Ltd |
| Nikon Inverted Microscope | Beijing Hengsanjiang Instrument Sale Ltd |
| AIRTECH Cleaning Working Table | Suzhou Antai Air Technology Ltd of Sujing Group |
| Eppendorf Centrifuge | Shanghai Kernel Biotech Co., Ltd |
| Sartorius Electronic Analysis Scale | Sartorius Scientific Instruments (Beijing) Ltd |
| Nikon Fluorescent Microscope | Shanghai Zeshi Opto-Electronic Technology Ltd |
| Mindray Routine Blood Analysis Meter | Shenzhen Mindray Biomedical Electronics Co., Ltd |
| Embedding Machine | Zhejiang Jinhua Kedi Instrumental Equipment Co., Ltd |

(continued)

| Names | Manufacturers |
|---|---|
| Pathological Slicing Machine | Zhejiang Jinhua Kedi Instrumental Equipment Co., Ltd |
| Slide Spreading Machine | Zhejiang Jinhua Kedi Instrumental Equipment Co., Ltd |
| Freezing Table | Zhejiang Jinhua Kedi Instrumental Equipment Co., Ltd |
| Oven | Shanghai Jinghong Experimental Instrument Co., Ltd |
| Glass Slide and Cover Glass | Jiangsu Sito Test Instrument Co., Ltd |
| Upright Optical Microscope | Olympus, Japan |
| Image System | Mshot |

**1.2 Reagents**

| Names | Manufacturers |
|---|---|
| Chloral hydrate | Tianjin Kemiou Chemicals Ltd |
| Physiological saline | SJZ No. 4 Pharmaceutical |
| Rat-IL-6 ELISA Test Kit | Tianjin Anorikang biotechnological Ltd |
| Rat-IL-1$\beta$ ELISA Test Kit | Tianjin Anorikang biotechnological Ltd |
| Rat-TNF-$\alpha$ ELISA Test Kit | Tianjin Anorikang biotechnological Ltd |
| NaCl | Tianjin Bohua chemical reagents Co., Ltd |
| Peptone | Solarbio |
| Yeast extract | Solarbio |
| 10% neutral buffered formalin fixing solution | Zhejiang Jinhuatonghe Biotechnology Co., Ltd, |

**1.3 Test medicaments**

The inventive pharmaceutical composition samples (prepared in Preparation Example 3)

**1.4 Negative Medicaments**

Amoxicillin capsules, Shandong Ziboxinda pharmaceutical Ltd.; batch number: 191040, in the valid period; specification: 0.25 g/tablet, usage and dose: referring to clinical usages; starting administration at the first day of modelling in a dose that is equivalent to clinical doses, each rat ig 0.36 g/kg, 1 time per day for 5 consecutive days.

**1.5 Test Animals**

SPF-grade SD rats, not being limited to female or male, with a weight of 180-220 g; provided by provided by Beijing Huafukang Biotechnology Ltd; certificate number: 1103222011009608; license number: SCXK (Jing) 2019-0008; feeding conditions: ABSL-2 laboratory, -20 Pa, temperature 22$\pm$1 °C and humidity 40-50%.

**1.6 Staphylococcus aureus**

Staphylococcus aureus standard cell lines 6538, commercially available from ATCC.

**1.7 Dose designs and medicament formulation**

1.7.1 Extracts of the inventive pharmaceutical compositions: clinical doses for human were 9 g/70 kg/d, and the dose for test rates were 3.5 g/kg/time, equivalent to 16 times of the clinical dose.

Formulations of medicinal solutions: before the test, distilled water was used to formulate a medicinal solution with a concentration of 0.35 g/ml, which was intragastrically administrated in 1 ml/100 g body weight/time, 2 times/day, and on 7th day after pre-administrations, modelling was started and the intragastric administration was continued for 5 days.

1.7.2 Amoxicillin capsules: referring to clinical usages. On 1st day, at 1 h after Staphylococcus aureus infection, the positive medicinal group was intragastrically administrated at a dose of 0.36 g/kg (equivalently equal to the clinical dose of a human), 1 time/day for 5 consecutive days.

Formulations of medicinal solutions: before the test, the granules inside capsules were formulated with distilled water into a medicinal solution with a concentration of 36 mg/ml, and stored at 4 °C for use.

### 1.8 Bacteria Culture

Two 250 ml conical flasks were taken, and to each of them, 1 g of yeast extract, 2 g of peptone, 2 g of sodium chloride and 200 ml of deionized water were added and mixed uniformly, to formulate LB culture media which were placed in a 4 °C chromatography cabinet at 4 °C and stored for use.

Staphylococcus aureus colonies were inoculated into a conical flask charged with the LB culture medium and cultured by shaking at 37 °C for 17 hours. The bacterial solution was centrifuged at 3220 rcf for 1 min, and by discarding the supernatant, it was counted under a microscope; the density of the bacterial solution was adjusted to $1.91\times10^9$/ml.

### 1.9 Grouping and Detection

28 SD rats were randomly divided into a normal group, a model group, an inventive pharmaceutical composition administrated group and an amoxicillin group, each group with 7 SD rats.

The group administrated with the pharmaceutical composition of the invention was is pre-administered in an intragastric route for seven days, at a dose of 3.5 g/kg, and twice per day; after 7 days, the other individual groups were modelled by injecting a Staphylococcus aureus bacterial solution on the pharynx, $1\times10^8$/rat for 2 consecutive days, and meanwhile, the administration was continued; for the amoxicillin group, the intragastric administration was started on the first day of modelling, at an administration dose of 0.36 g/kg, once per day; after the modelling was finished, the inventive pharmaceutical composition administrated group and the amoxicillin group were continuously administered for three days, and on 4th day, they were weighed and dissected for observation and detection.

### 1.9.1 Scoring of Pharyngeal Lesions

With scoring standards (from Standards for Preparation of Animal Models Suffered from Acute Pharyngitis, enacted by Traditional Chinese medicine Experimental Pharmacology Professional Committee of Traditional Chinese Medicine Institute, Traditional Chinese Medicine Pharmacology and Clinics 2018; 34 (1)), according to color, gloss, secreted substances of tissues, and degrees of pharyngeal congestions and swelling, the lesions were classified into 4 grades.

"-": pharyngeal tissues were in light red color, and they had moist and glossy surfaces, and no secreted substances, no congestion, no swelling and other pathological phenomena;

"+": the mucosa in the pharynx had poor gloss, and there were a small amount of secreted substances, and slight congestions and swelling;

"++": the mucosa in the pharynx was in a dark red color and poor in the gloss, and secreted substances exist therein, accompanying by moderate congestions and swelling and other phenomena;

"+++": the mucosa in the pharynx exhibited a dark red color, increased secretion of mucus, and evident congestion and swelling.

### 1.9.2 Characterizations and Scoring for Appearance Behavior of rats

With scoring standard (from Standards for Preparation of Animal Models Suffered from Acute Pharyngitis, enacted by Traditional Chinese medicine Experimental Pharmacology Professional Committee of Traditional Chinese Medicine Institute, Traditional Chinese Medicine Pharmacology and Clinics 2018; 34 (1)), according to weights, mobility, fur color and gloss of rats, they were classified into 4 grades, as shown in the following Table 4.

Table 4

| Index grades | weight | Mobility | Fur gloss |
|---|---|---|---|
| 0 | Normal | Normal | Normal |
| 1 | Substantially normal | Substantially normal | Substantially normal |
| 2 | Slight decrease | Slight decrease | Slight decrease |
| 3 | Evident decrease | Evident decrease | Evident decrease |

**1.9.3 Detections for counting of white blood cells of rats and counting classifications thereof**

Before each group of animals were scarified, blood was taken by cutting tails and smeared on plates to conduct routine blood examinations about number of white blood cells, lymphocyte percentages and neutrophils percentages.

**1.9.4 Pathological Detections and Observations**

**1.9.4.1 Paraffin-Embedded Slides of Tissue**

Material taking: rats were anesthetized and pharyngeal tissues were taken and fixed in 4% paraformaldehyde for more than 24 h. The tissues were taken from the fixing solution and in a fume hood, the tissues at the target region were trimmed with a surgical knife. The trimmed tissues and the corresponding tags were placed in a dehydration box.
Dehydration: the dehydration was conducted by placing the dehydration box in a hanging basket, and in a dehydration machine, the dehydration was conducted with alcohols with gradient concentrations in order. 75% alcohol 1 h - 85% alcohol 1 h - 90% alcohol 1 h - 95% alcohol 1 h - anhydrous ethanol I 30 min - anhydrous ethanol II 30 min - alcohol benzene 5-10 min - xylene I 5-10 min - xylene II 5-10 min - wax I 1 h - wax II 1 h - wax III 1 h.
Embedding: the tissues immersed with wax were embedded in an embedding machine. The molten wax was placed in an embedding frame first, and before it was solidified, the tissues were taken from the dehydration box and placed in the embedding frame according to requirements on embedded faces and corresponding tags were attached. They were cooled on a freezing table at -20 °C, and after wax solidification, the wax block was taken from the embedding frame and trimmed.
Slicing: the trimmed wax block was sliced on a paraffin slicing machine, in a thickness of 4 $\mu$m. The slices were floated on a slide spreading machine at 40 °C to spread the tissues, and a slide glass were used to take tissues and baked at an oven at 60 °C. After water was baked to dryness and the wax to melt, the slide glass was taken and stored at normal temperature for use.

**1.9.4.2 HE Staining**

The paraffin slides were dewaxed into water; the slides, by being placing in order in xylene I30 min - xylene II 30 min - anhydrous ethanol I 10 min - anhydrous ethanol II 10 min - 95% alcohol 5 min - 90% alcohol 5 min - 80% alcohol 5 min - 70% alcohol 5 min - distilled water, were washed.
Staining nuclei with Hematoxylin: slides were placed in Harris hematoxylin and stained for 5-10 min, and they were washed with tap water. After differentiating with 1% hydrochloric acid and alcohol for several seconds, they were washed with tap water for 10 min, returned to blue color with PBS for 5 min, and washed with running water.
Staining cytoplasm with Eosin: slides were placed in an Eosin solution and stained for 1-3 min.
Dehydration and sealing of slides: slices, by being placing in 95% alcohol I5 min - 95% alcohol II 5 min - anhydrous ethanol I5 min - anhydrous ethanol II 5 min - xylene I5 min - xylene II 5 min in order, were dehydrated to transparent, and the sides were taken out of xylene, slightly dried in air and sealed with neutral gum.
A microscope is used for detections and the images were collected for analysis.

**1.9.5 Detection of Inflammatory Factors in Rat Serum**

Rates were anesthetized. Blood was taken from abdominal aorta and injected into a procoagulant tube, standing for 45 min at room temperature, and it was centrifuged at 3500 rpm for 10 min. The supernatant was suction taken into a sterile EP tube and stored at -80 °C. Before measurements, the samples were re-thawed at room temperature, and the IL-1$\beta$, IL-6 and TNF-$\alpha$ levels in serum were measured by enzyme-linked immunosorbent assay (ELISA). The operations were carried out according to the instruction of the kit, and each index was detected by an enzyme-labelled meter at the absorbance 450 nm.

**2 Experimental Results**

**2.1 Impacts of the inventive pharmaceutical compositions on appearance behaviors and pharyngeal scores of rats suffered from acute pharyngitis**

The results were shown in Tables 5 and 6.

Table 5: Impacts of the inventive pharmaceutical compositions on appearance behavior characterizations of rats suffered from acute pharyngitis ($\bar{x}\pm$s, n=7)

| Groups | Number of animals | Doses (g/kg) | Weight | Mobility | Fur gloss | Thymus gland (g) |
|---|---|---|---|---|---|---|
| Normal group | 7 | - | 0.00±0.00 | 0.00±0.00 | 0.00±0.00 | 0.55±0.11 |
| Model group | 7 | - | 2.43±0.53** | 2.43±0.53** | 2.57±0.53** | 0.20±0.05** |
| Group of pharmaceu tical compositio ns of the invention | 7 | 3.5 | 1.57±1.13 | 1.71±0.49# | 1.71±0.49## | 0.21±0.04 |
| Amoxicillin group | 7 | 0.36 | 0.71±1.25## | 1.43±0.53## | 1.14±0.38## | 0.26±0.11 |
| Note: in comparison to normal group **P<0.01; in comparison to model group, #P<0.05, ##P<0.01. | | | | | | |

Table 6: Impacts of the inventive pharmaceutical compositions on pharyngeal lesions of rats suffered from acute pharyngitis

| Groups | Number of animals | Doses (g/kg) | - | + | ++ | +++ |
|---|---|---|---|---|---|---|
| Normal group | 7 | - | 7 | 0 | 0 | 0 |
| Model group | 7 | - | 0 | 0 | 2 | 5 |
| Group of pharmaceutical compositions of the invention | 7 | 3.5 | 0 | 7 | 0 | 0 |
| Amoxicillin group | 7 | 0.36 | 0 | 6 | 1 | 0 |

As shown by the results in Table 5, in comparison to the normal group, the weights of the rats in the model group were evidently reduced (P<0.01); the weights of thymus gland significantly decreased (P<0.01); in the model group, a phenomenon that animals were crowed and still was exhibited, and they exhibited bad mental states and evidently reduced mobility (P<0.01); the animals in the model group has dry and dull fur, and the scores for fur gloss were significantly increased (P<0.01).

In comparison to the rates in the model group, the rats in the inventive pharmaceutical composition administrated group had increased weights and thymus gland weights, obviously increased mobility, which are consistent with the characterizations for the behaviors of the rats in the amoxicillin group.

As shown by the results in Table 6, the pharyngeal inflammatory responses of the rats in the normal group were all in a "-" grade, and no inflammatory features appear. The model group had the pharyngeal inflammatory response in the grade "++" and the grade "+++" and evident lesions in pharyngeal mucosa and tissues, and thus it was successfully modelled. A majority of the animals in the inventive pharmaceutical composition administrated group were accompanied with small quantities of secreted substances, and slight congestion and swelling, and inflammatory symptoms were evidently improved, exhibiting the grade "+", occasionally the grades "-" and "++". Animals administrated with amoxicillin were recovered consistently with recovery conditions of pharyngeal lesions of the rats in the inventive pharmaceutical composition administrated group, most exhibiting the grade "+" and occasionally the grade "++".

**2.2 Impacts of the inventive pharmaceutical compositions on peripheral white cell counts of rates suffered from acute pharyngitis and on classifications thereof**

The results were shown in the following Table 7.

Table 7: Impacts of the inventive pharmaceutical compositions on peripheral white cell counts of rates suffered from acute pharyngitis and classifications ($\bar{x}\pm$s, n=7)

| Groups | Number of animals | Doses (g/kg) | WBC ($\times 10^9$/L) | L (%) | N (%) |
|---|---|---|---|---|---|
| Normal group | 7 | - | 7.38±1.1 | 72.85±2.53 | 23.4±2.25 |
| Model group | 7 | - | 9.23±3.84 | 55.2±5.4** | 40.91±5.3** |
| Group of pharmaceutical compositions of the invention | 7 | 3.5 | 4.33±2# | 72.44±10.06## | 22.87±9.49## |
| Amoxicillin group | 7 | 0.36 | 8.17±30 | 76.37±7.42## | 19.5±6.77## |

Note: WBC ($\times 10^9$/L): white cells; L (%): percentages of lymphocytes; N (%): percentages of neutrophils. In comparison to normal group **P<0.01; in comparison to model group, #P<0.05, ##P<0.01.

As shown by the results in Table 7, in comparison to the normal group, the number of white cells of the rat acute pharyngitis model group was increased (P<0.05), the lymphocyte percentages were evidently reduced, and the neutrophil percentages were evidently increased (P<0.01); in comparison to the model group, the lymphocyte percentages of the rats in the inventive pharmaceutical composition group and the amoxicillin group were increased (P<0.01), and the neutrophil percentages were reduced (P<0.01).

**2.3 Impacts of the inventive pharmaceutical compositions on pathological morphology of pharyngeal tissues of rates suffered from acute pharyngitis**

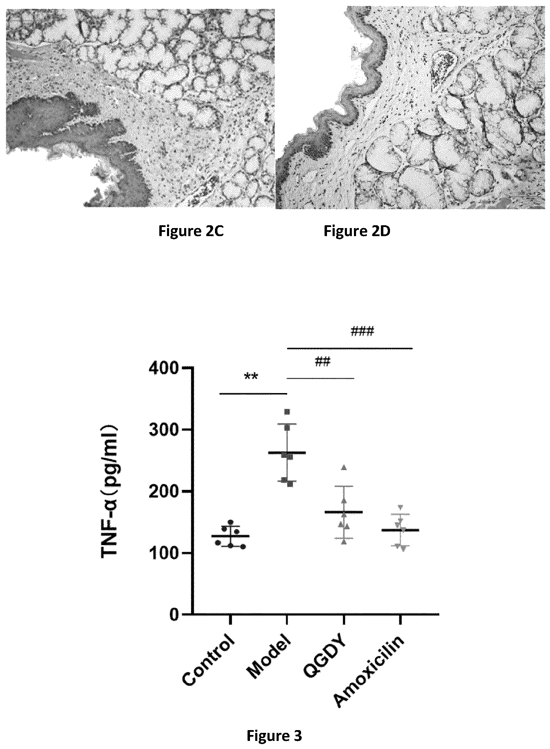

As shown in Figs. 2A-2D, in pharyngeal tissues of the rats in the normal group, there were clear structures, smooth mucous epithelium and no thickening; the laminae propria was scattered in lymphatic tissues and glands, the glands were normal in shape; there were a small amount of small blood vessels under the mucous, no granulation tissue existed, and the fibers of the muscular layer were arranged closely. In the pharyngeal tissues of the rats in the model group, there were unclear boundaries, keratinized mucous epithelium, and an epithelial exfoliation phenomenon; blood vessels under the lamina propria and the mucous were increased and congested, and the infiltrations of a large amount of inflammatory cells were observed; the gland walls, in comparison to the normal group, were evidently thickened; most epithelial cells of mucous gland were necrotic, and the growth of a large amount of granulation tissues growth was observed; the fibers of the muscular layer were arranged loosely. In pharyngeal tissues of the rats in the inventive pharmaceutical composition administrated group, the mucous epithelium was gradually recovered into smoothness, the gland wall recovered to a normal level, the infiltration degrees of inflammatory cells on the mucosa and under the mucosa were evidently reduced, and the fibers of the muscular layer were arranged tightly. The recovered conditions of the pharyngeal mucosa of the rats in the amoxicillin group were very similar to those in the inventive pharmaceutical composition group, indicating that after administration, the pharyngeal pathological conditions were improved.

**2.4 Impacts of the inventive pharmaceutical compositions on contents of inflammatory factors in peripheral blood of rats suffered from acute pharyngitis**

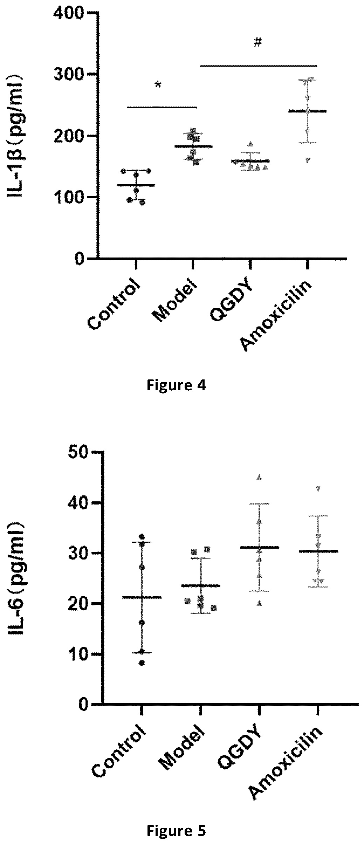

The results are shown in the following Table 8 and in Figs 3, 4, 5.

Table 8: Impacts of the inventive pharmaceutical compositions on contents of inflammatory factors in peripheral blood of rats suffered from acute pharyngitis ($\bar{x}\pm$s, n=7)

| Groups | Number of animals | Doses (g/kg) | TNF-$\alpha$ (pg/mL) | IL-1$\beta$ (pg/mL) | IL-6 (pg/mL) |
|---|---|---|---|---|---|
| Normal group | 7 | - | 121.13±22.23 | 124.01±24.39 | 24.88±13.88 |
| Model group | 7 | - | 253.27±49.47** | 189.95±26.58** | 21.50±7.30 |
| Group of pharmaceutical compositions of the invention | 7 | 3.5 | 180.26±53.59# | 169.1±30.6 | 27.22±13.06 |

(continued)

| Groups | Number of animals | Doses (g/kg) | TNF-$\alpha$ (pg/mL) | IL-1$\beta$ (pg/mL) | IL-6 (pg/mL) |
|---|---|---|---|---|---|
| Amoxicillin group | 7 | 0.36 | 145.14$\pm$30.98## | 254.04$\pm$59.15# | 28.21$\pm$8.67 |
| Note: in comparison to normal group **P<0.01; in comparison to model group, #P<0.05, ##P<0.01. | | | | | |

As shown in the results of Table 8, in comparison to the normal group, the contents of the inflammatory factors TNF-$\alpha$ and IL-1$\beta$ in peripheral blood of the rats in acute pharyngitis model were significantly increased (P<0.01), and the content of the IL-6 was reduced; in comparison to the model group, the inventive pharmaceutical composition group and the amoxicillin group can inhibit the expressions of TNF-$\alpha$ and IL-1$\beta$ (P<0.01) in peripheral blood of rats.

## 3. Brief Summary

The inventive pharmaceutical compositions can significantly improve lesion conditions of the pharyngeal mucosa of rats suffered with acute pharyngitis, increase the weights and the thymus gland weights of rats, improve the mobility of the rats, improve the fur states of rats, inhibit the increases of the number of white blood cells of the rats suffered with acute pharyngitis, the increase of the lymphocyte percentages, and reductions of the neutrophil percentages, and relieve the pathological lesion degrees of the pharyngeal tissues of the rats after infections, and it can also significantly reduce the content of TNF-$\alpha$ in the serum of rats suffered with acute pharyngitis. This indicates that: the inventive pharmaceutical compositions have evident effects for preventing and treating a rat acute pharyngitis model by staphylococcus aureus, thereby providing laboratory bases for clinical medicament administrations of the acute pharyngitis.

Experimental Example 3: Studies on effects of the inventive pharmaceutical compositions on the expression of LPS-induced THP-1 inflammatory factor

The experiment studied the impacts of the inventive pharmaceutical compositions on the releases of LPS-induced cytokine TNF-$\alpha$, IP-10, IkB$\alpha$, IL-6 and IL-1$\beta$ of human monocyte THP-1 and preliminarily discusses the anti-inflammatory effects of the inventive pharmaceutical compositions.

**1 Materials and Methods**

**1.1 Experimental Instruments**

| Instrument names | Manufacturers |
|---|---|
| Sartorius electronic analytical scale | Sartorius Scientific Instruments (Beijing) Ltd |
| Thermo Fisher high speed freezing centrifuge | Saimer Feishire Science and Technology Co., Ltd |
| INFORS desk-type shaking incubator | Infers (China) Biotechnology Ltd |
| Nikon inverted microscope | Beijing Hengsan Jiang Instrument sales Ltd |
| TECAN enzyme-labelled meter | Beijing Haitianyoucheng Science and Technology Ltd |
| RNA concentration measuring instrument | Saimer Feishire Science and Technology Co., Ltd |
| Eppendorf temperature-constant mixing instrument | Eppendorf Co., German |
| Bio-Rad real-time quantitative PCR instrument | Bio-Rad Co., USA |
| Eppendorf Minimal desk-type high-speed freezing centrifuge | Eppendorf Co., German |
| Haier biological safety cabinet | Qingdao Haier Special Appliances Co., Ltd |
| Thermo $CO_2$ cell incubator | Saimer Feishire Science and Technology Co., Ltd |

**1.2 Reagents**

| Reagent name | Manufacturers |
|---|---|
| Lipopolysaccharide (LPS) | Sigma, USA |
| CCK-8 test kit | Suzhou Yuchang Biotechnology Ltd |
| LDH test kit | Dojindo Lab, Japan |
| CWBIO ultra-pure RNA extraction test kit | Kangwei Century Biotechnology Ltd |
| TransScript reverse transcription test kit | Beijing Quanshijin Biotechnology Ltd |
| Promega qPCR test kit | Promega Co. |
| Fetal bovine serum | Biological Industries, Israel |
| RPMI culture solution | |
| Trypsase | |

**1.3 Experimental Medicaments**

The inventive pharmaceutical compositions (prepared in Preparation Example 3).

**1.3.1 Dissolutions of the inventive pharmaceutical compositions**

2.5 mg of the inventive pharmaceutical compositions were precisely weighed into 1 ml of double distilled water to obtain 2.5 mg/ml of a standard stock solution of the inventive pharmaceutical compositions, and by the means of a gradient dilutions with double distilled water, the sample solutions at 2.5 mg/ml, 1 mg/ml and 0.5 mg/ml were formulated.

**1.3.2 Dissolutions of LPS**

1 mg of LPS was quickly centrifuged at less than 21 °C in a centrifuge and sufficiently dissolved by adding 1 mL of $ddH_2O$. After filtering the solution by using a filter, it was sub-packaged to facilitate use in experimental processes, and stored at the temperature -20 °C.

**1.4 Cell Culture**

**1.4.1 Passages of THP-1 Cells**

THP-1 cells used in the experiment were purchased from ATCC, being suspended cells, and thus upon passages, they were not in need of centrifugation. 20 mL of a RPMI cell culture solution containing 10% heat-inactivated fetal bovine serum and 1% bispecific antibodies were added into a 75 $cm^2$ cell culture flask, and further added with 10 mL of cell solution from the original 25 $cm^2$ culture flask, and they were placed into a temperature-constant $CO_2$ cell incubator at 37 °C for continuous culture.

**1.4.2 Cryopreservation of THP-1 cells**

THP-1 cells were centrifuged at 800 rmp for 5 minutes and the supernatant was discarded. An appreciate amount of a cell cryopreserved liquid was taken according to actual situations, to precipitate cells which were rapidly re-suspended, and injected into a cryopreservation tube according to 1 mL/tube. The cryopreservation tube was placed in an isopropanol cryopreservation box, and after a gradient temperature decrease at -80 °C, the cells were transferred in a liquid nitrogen tank for storage.

**1.4.3 Resuscitation of THP-1 cells**

The cryopreservation tube was taken from liquid nitrogen, rapidly placed in a 37 °C water bath, and shaken quickly to thaw the cells. The cells were removed from the cryopreservation tube and added with 4 mL of the culture solution, and after being mixed uniformly, they were centrifuged for 5 minutes at 800 rpm. The supernatant was discarded, and the cell precipitates were re-suspended with 4 mL of the culture solution and then added with the culture solution to get a volume of 10 mL, and they were added to a 25 cm$^2$ culture flask which was placed in a temperature-constant $CO_2$ cell incubator at 37 °C where cells were cultured for 3-4 days. When the number of the cells reaches $1\times10^6$ per milliliter, the passage culture may be conducted.

### 1.4.4 Counting of THP-1 cells

A clear cell counting plate was taken and covered with a cover glass, and 10 μL of a uniformly dispersed cell suspension were added from the edge between the cover glass and the counting plate so as to fill the space between the counting plate and the cover glass. The plate was observed under an inverted microscope, to calculate the number of the cells in the four grid areas. For cells on the lines, only the cells on the left lines and on the right lines were counted, and clustered cells are counted as a single cell.

The density of cells was calculated according to the equation:

$$\text{Cell numbers/mL} = (\text{cell numbers in four grid areas})/4\times10^4\times\text{dilution time}$$

### 1.5 Detections on Cell Viability

The experiment uses CCK-8 and LDH test kits to investigate impacts of the pharmaceutical compositions of the invention with different concentrations on the viability of THP-1 cells.

The THP-1 cells in the logarithmic growth phase were taken and inoculated into a 96-well cell culture plate at a density of $1\times10^6$ cell/mL, each well with 100 μL. 2.5-0.5 μg/mL of the inventive pharmaceutical compositions were respectively added into the cell culture wells, each well with 1 μL and each concentration with 6 compound wells. Meanwhile, a control group without any medicaments was configured and subjected to CCK8 and LDH respectively. After 24 h cultures, the supernatant was suction taken and placed in the other new 96-well plate, and the absorbance (OD) was measured at 490 nm according to the operations of the LDH test kit. 10 μL of the CCK-8 solution were added to each well of the other 96-well plate, and incubated at 37 °C for 1 hour, and the absorbance (OD value) was measured by setting an enzyme-labelled meter at 450 nm.

### 1.6 Establishment of an LPS-induced THP-1 cell inflammatory model

THP-1 cells were incubated in a 6-well plate at a density of $1\times10^6$ cells/mL and after 24 h, the cells were treated in groups. In a model group, the LPS with a final concentration of 100 ng/mL was added; in the control group, the LPS was replaced with double distilled water; in the medicament-administrated group, the culture medium, in addition to the LPS, was further added the extracts of the inventive pharmaceutical compositions with final concentrations 25 μg/ml, 10 μg/ml and 5 μg/ml respectively. Cells were collected for measurements after they were pre-stimulated with the inventive pharmaceutical compositions for 4 hours and then stimulated by adding the LPS for 16 hours.

### 1.7 Extraction of Cell RNA

(1) The treated cell suspension was collected in a 15 mL centrifuge tube, and centrifuged at 1000 rmp for 3 minutes, the supernatant was discarded, and the cells were collected.

(2) After the cell precipitates were added with 1 ml of TRIzon Reagent, they were repeatedly brown and tapped several times so that the sample was fully split. The sample was left at room temperature for 5 minutes to completely separate protein-nucleic acid complexes.

(3) Chloroform was added at a proportion of 200 μl of chloroform relative to 1 ml of TRIzon Reagent, and they were sealed with the tube cap. After the tube was shaken vigorously for 15 seconds, it was left at room temperature for 2 minutes.

(4) Centrifugation was conducted at 12,000 rpm for 10 minutes at 4 °C, and at this time, the sample was divided into three layers: a red organic phase, a middle layer and an upper colorless aqueous phase. The RNAs were mainly in the upper aqueous phase which was transferred to a new RNase Free centrifuge tube (provided for oneself).

(5) To the resulting aqueous solution, an equal volume of 70% ethanol (formulated without RNase water) was added, and they were mixed uniformly by inversion shaking. All the solution obtained in the previous in the last step was added to an adsorption column (Spin Columns RM) which had been loaded into a collection tube. If the solution cannot be added completely at one time, it can be transferred for multiple times.

(6) After the solution was centrifuged at 12,000 rpm for 20 seconds, the waste liquid was discarded, and the adsorption column was placed back into the collection tube again.

(7) After 700 $\mu$L of Buffer RW1 were added to the adsorption column and centrifuged at 12,000 rpm for 20 seconds, the waste liquid in the collection tube was discarded, and the adsorption column was placed back in the collection tube again.

(8) After 500 $\mu$L of Buffer RW2 (prior to use, it was checked if anhydrous ethanol has been added) were added to the adsorption column and centrifuged at 12,000 rpm for 20 seconds, the waste liquid in the collection tube was discarded, and the adsorption column was placed back in the collection tube again

The step (8) was repeated.

(9) After the solution were centrifuged at 12,000 rpm for 2 minutes, the waste liquid in the collection tube was discarded, and the adsorption column was placed at room temperatures for several minutes, to air dry it thoroughly.

(10) The adsorption column was placed in a new RNase-free centrifuge tube, and 30-50 $\mu$l of RNase-free Water were added to the middle portion of the adsorption column; the solution was left at room temperature for 1 minute and centrifuged at 12,000 rpm for 1 minute. A RNA solution was collected, and the RNA was stored at -70 °C to prevent degradations.

(11) The RNA concentrations were measured by using an RNA concentration measurement meter, and 1.5 $\mu$L of the RNA solution were taken and its concentration was measured, with the RNase-Free Water as a blank control. A total RNA concentration (ng/$\mu$L) as well as values of OD260/280 and OD 260/230 were read on an RNA concentration measurement meter, respectively.

### 1.8 RNA Reverse Transcription and PCR Real-Time Quantitation

### 1.8.1 RNA Reverse Transcription

The following operations were conducted on ice, and according to Table 9, 20 $\mu$L of a reaction system was formulated and vortexed and shaken vigorously. After the reaction system was mixed uniformly, it was centrifuged briefly to collect the solution on the side wall to the bottom of the tube, and then incubated at 42 °C for 15 minutes and 85 °C for 5 seconds. Upon completion, the sample was cooled by placing on ice.

Table 9: Reverse Transcription System

| Reagent name | 20 $\mu$l reaction system |
|---|---|
| Total RNA/mRNA | $\leq$1 $\mu$g/$\leq$100 ng |
| 5$\times$TransScript All-in-One No-RT vs SuperMix for qPCR | 4 $\mu$L |
| gDNA Remover | 1 $\mu$L |
| RNase-free Water | Variable |
| Total volume | 20 $\mu$L |

### 1.8.2 PCR Real-time Quantitation

On ice, 20 $\mu$L of a PCR reaction system was formulated according to the prescriptions listed in Table 10 by using a real-time quantitative PCR test kit. After the formulation was completed, the reaction system was vortexed so that it was fully and uniformly mixed. After centrifugation, a real-time quantitative PCR experiment was conducted. In this experiment, the reaction and cycle conditions of the real-time quantitative PR-PCR were set below: 95 °C 10 s, 60 °C 30 s, totally cycling 40 times. A melting curve was added at temperatures between 50 °C and 99 °C. The sequences of the primers involved in the experiment were shown in Table 11. After detections, the Ct value was obtained, and a $\Delta$Ct was the difference the Ct value of genes to be tested and the Ct value of internal reference genes. By analyzing the data of the reaction plate, the corresponding Ct value of each sample, in each reaction, was obtained, and by making calculations, the $\Delta$Ct value was obtained. After making comparisons to the control group, the values of $2^{-\Delta\Delta Ct}$ were taken for making statistics.

Table 10: PCR Reaction system

| Reagent names | 20 $\mu$L reaction system |
|---|---|
| 2xqPCR Master Mix | 10 $\mu$L |
| Upstream primer | 0.5 $\mu$L |
| Downstream primer | 0.5 $\mu$L |
| RNase-Free Water | 8 $\mu$L |
| cDNA products | 1 $\mu$L |

Table 11: Sequences of experiment-related primers

| Primers | Upstream primers | Downstream primers |
|---|---|---|
| IP-10 | GGTGAGAAGAGATGTCTGAATC (SEQ ID NO: 1) | GTAGGGAAGTGATGGGAGAG (SEQ ID NO: 2) |
| IL-6 | GGCACTGGCAGAAAACAACC (SEQ ID NO: 3) | GCAAGTCTCCTCATTGAATCC (SEQ ID NO: 4) |
| I$\kappa$B$\alpha$ | GCACCTCCACTCCATCCTGAAGG (SEQ ID NO: 5) | CCATTACAGGGCTCCTGAGCATTG (SEQ ID NO: 6) |
| IL-1$\beta$ | CCAGGGACAGGTATGGAGCA (SEQ ID NO: 7) | TTCAACACGCAGGACAGGTACAG (SEQ ID NO: 8) |
| TNF-$\alpha$ | GCGGTGCTTGTTCCTCAG (SEQ ID NO: 9) | GGCTACAGGCTTGTCACTC (SEQ ID NO: 10) |
| GAPDH | ATGATTCTACCCACGGCAAG (SEQ ID NO: 11) | CTGGAAGATGGTGATGGGTT (SEQ ID NO: 12) |

### 1.9 Statistical Methods

Experimental data was subjected to single-way ANOVA analyses by using SPSS 20.0 software to assess the differences between the means of the data. In the case of $P<0.05$, it was contemplated that the data had statistical differences, and the data was expressed as means $\pm$ S.E.m.

### 2 Experimental Results

### 2.1 Screening of non-toxic doses of the inventive pharmaceutical compositions for THP-1 cells

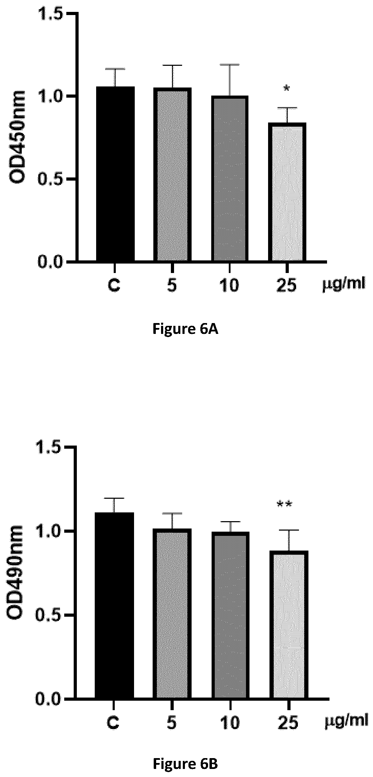

CCK-8 and LDH methods were used for detecting impacts of the inventive pharmaceutical compositions on the viabilities of the THP-1 cells at 25 $\mu$g/ml, 10 $\mu$g/ml and 5 $\mu$g/ml. The results showed that the viabilities of the THP-1 cells in the inventive pharmaceutical composition group at 5 $\mu$g/ml and 10 $\mu$g/ml had no significant difference as compared with those in a normal control group. Safety concentrations of medicaments were utilized to investigate the anti-inflammatory effects of the inventive pharmaceutical compositions (Fig. 6A-6B).

### 2.2 Inhibitory effects on TNF-$\alpha$, IP-10, IKB$\alpha$, IL-6 and IL-1$\beta$ secretions of THP-1 cells

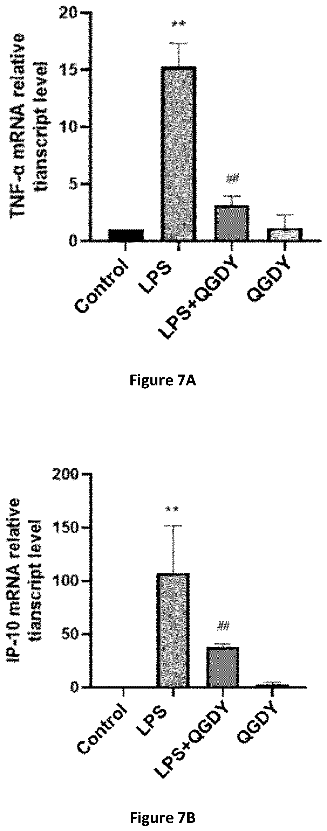

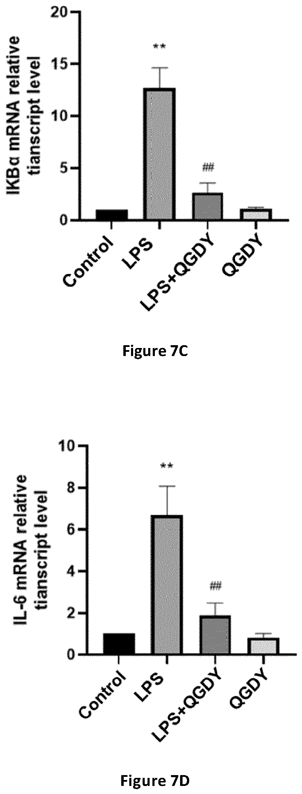

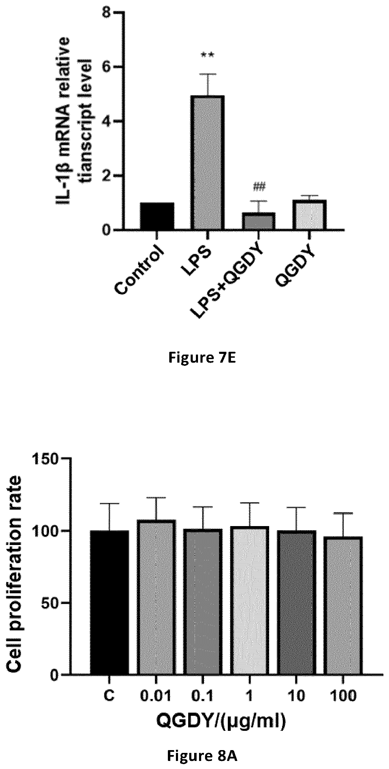

In comparison to the control group, the LPS stimulation evidently induced a significant increase in mRNA expressions of TNF-$\alpha$, IP-10, IKB$\alpha$, IL-6 and IL-1$\beta$ of THP-1 cells; in comparison to the LPS-stimulated group, 10 $\mu$g/ml of the inventive pharmaceutical compositions could significantly inhibit mRNA expressions of TNF-$\alpha$, IP-10, IKB$\alpha$, IL-6 and IL-1$\beta$ of inflammatory THP-1 cells, indicating that the inventive pharmaceutical compositions could inhibit the release of LPS-induced cytokine (FIG. 7A-FIG. 7E).

### 3 Brief Summary

The inventive pharmaceutical compositions can significantly inhibit the releases of cytokines TNF-$\alpha$, IL-6 and IL-1$\beta$ of LPS-induced THP-1 cells, indicating anti-inflammatory effects.

Experimental Example 4: Experimental studies on anti-oxidation/anti-inflammatory pharmacodynamics of the inventive pharmaceutical compositions

This experiment, based on a luciferase reporter gene system of ARE and NF-kB, investigated impacts of the inventive pharmaceutical compositions on the activities of the ARE and NF-kB, and preliminarily discussed the autoxidation and anti-inflammatory effects of the inventive pharmaceutical compositions.

**1 Materials and Methods**

**1.1 Experimental Instruments**

| Experimental instruments | Manufactures |
|---|---|
| High-temperature high-pressure sterilizing pot | Shandong Boke Biochemical Industries, Ltd |
| FORMA3111 temperature-constant incubator | Saimer Feishire Science and Technology Co., Ltd |
| Low-temperature high-speed centrifuge | Saimer Feishire Science and Technology Co., Ltd |
| Infinite M200 multifunctional enzyme-labelled meter | Swiss Diken Co Ltd |
| VICTORTM X5 Multilabel Functional Plate Reader | Perkins Elmer Co., USA |
| Biological Safety Cabinet | Haier Group Co. |

**1.2 Reagents**

| Names | Manufacturers |
|---|---|
| Firefly luciferase reporter gene vectors (pGL4.37, pGL4.32, pGL4.75) | Promega Co., USA |
| Ampicillin | Beijing Solarbia Co. |
| LB liquid medium (Dry powder) | Beijing Solarbia Co. |
| LB solid medium (Dry powder) | Beijing Solarbia Co. |
| Glycerol | Beijing Solarbia Co. |
| Plasmid Mini-extraction test kit | Tiangen Biochemical technology Ltd |
| DMEM culture medium | Gibco Corp, USA |
| Penicillin-streptomycin | Gibco Corp, USA |

**1.3 Test Medicaments**

A inventive pharmaceutical composition according to prescription was soaked and extracted twice, for every time, soaking for 40 minutes with boiling water in an quantity of 10 times thereof, and it was filtered and concentrated at a temperature below 60 °C to a sugar degree of 15-17Brix and spray dried to obtain a medicament.

**1.4 Positive Medicaments**

Dexamethasone, MedChemExpress Co. Ltd; batch number: HY-14648, in a valid period; specification: 500 mg; usages and doses: referring to clinical usages, formulated into a 10 mM mother liquor.

tBHQ ((Tertiary butylhydroquinone), MedChemExpress Co. Ltd; batch number: HY-100489, in a valid period; specification: 500 mg; usages and doses: referring to clinical usages, formulated into a 100 mM mother liquor.

**1.5 Test cells**

293T cells, commercially available from ATCC cell bank.

**1.6 Competent Cell DH5a**

Competent cells DH5a, commercially available from Tiangen Biochemical technology Ltd.

**1.7 Dose Design and Medicament Formulations**

The inventive pharmaceutical compositions: 0.01 $\mu$g/mL, 0.1 $\mu$g/mL, 1 $\mu$g/mL, 10 $\mu$g/mL, 100 $\mu$g/mL.

Medicament Formulations: prior to test, a medicinal powder was formulated into a medicinal solution with the concentration of 100 mg/ml by distilled water, which was diluted into a test concentration in use.

**1.8 Plasmid Extraction**

**1.8.1 Preparation of LB Liquid Culture Medium**

After a triangular flask was sterilized at high temperatures and high pressures, 2.5 g of a dry powder of LB liquid culture medium were added into the flask, and fully dissolved by adding 100 mL of sterilized ultra-pure water. After attaching a filtration membrane on the opening of the flask, it was sterilized at high temperatures and high pressures. After the solution was cooled to about 55 °C, it was added with 100 $\mu$L of 100 mg/mL ampicillin, and they were uniformly mixed to obtain a culture medium. Cells were cultured in a DMEM culture medium containing 10% FBS, and the passage was started when the cells in the flask grown to 80%-90%.

**1.8.2 Preparation of LB Solid Culture Medium**

0.4 g of the dry powder of LB solid culture medium were taken. 0.3 g of the dry powder of LB solid culture medium were placed in a triangular flask which was sterilized at high temperatures and high pressures and added with 20 mL of sterilized ultrapure water, and they were heated to boil, dissolved by stirring and sterilized at high temperatures and high pressures. After the solution was cooled to 50-60 °C, 50 $\mu$L of ampicillin (50 $\mu$g/mL) were added and mixed uniformly to obtain the culture medium; the above solution was sub-packaged in culture dishes, and after it was solidified by cooling, the culture dishes were covered with covers, inverted and stored a refrigerator at 4 °C.

**1.8.3 Transformation and Verification of Plasmid Vectors**

After competent cells DH5a were gently mixed to be uniform, 100 $\mu$L of the cells were taken and placed in a 15 mL centrifuge tube which were added with 1 $\mu$L of plasmid pGL4.37 sample. The mixture was placed on ice for 30 minutes and immediately transferred into a water bath at 42 °C where it was incubated for 45 s, and then it was transferred on ice where it stayed for 1-2 min to finish the transformation of the competent cells. 100 $\mu$L of the above transformed competent cells were suction taken into an appropriate amount of the LB liquid culture medium, and mixed uniformly. By a sterile coater, an appropriate amount of the above bacterial solution was coated onto the LB solid culture medium which was placed at room temperature until the bacterial solution was fully absorbed. At 37 °C, the culture medium was inverted at 37 °C and cultured for 12 h.

In a biological safety cabinet, six bacterial granules were selected and added to six conical flasks containing appropriate amounts of the LB liquid culture medium respectively, to obtain six bacterial solution samples labelled with numbers 1 to 6. The above samples were kept at the temperature 37 °C, and after they were oscillated at 160 rpm for 12 hours, 1 ml of the samples with individual numbers were taken and placed in a 1 mL centrifuge tube, and they were sequenced in Huada Gene, to identify positive clones.

**1.8.4 Extractions and determinations of plasmid**

(1) 500 $\mu$L of an equilibration solution BL was added to an adsorption column CP3, and centrifuged at 12000 rpm for 1 min. The waste liquid was discarded from the collection tube, and the adsorption column was placed back in the collection tube again.

(2) 1-5 mL of an overnight cultured bacterial solution were taken and placed in a centrifuge tube, and the solution was centrifuged at 12000 rpm for 1 min. The supernatant was suction removed as much as possible. When the bacterial solution is too much, by multiple centrifugations, the bacteria were precipitated and collected in a centrifugal tube.

(3) 250 μl of a solution P1 were added to the centrifugal tube with bacteria precipitates, to thoroughly suspend the bacterial precipitates.

(4) 250 μL of a solution P2 was added to the centrifuge tube which was gently turned upside down for 6 to 8 times to sufficiently split the bacteria.

(5) 350 μl of a solution P3 were added into the centrifuge tube which was immediately and gently turned upside down for 6-8 times, and after mixing uniformly, and the solution was centrifuged at 12000 rpm for 10 min.

(6) The supernatant collected in the last step was transferred into an adsorption column CP3 by using a pipette and centrifuged at 12000 rpm for 30-60 s, and the waste liquid in the collection tube was discarded. The adsorption column CP3 was placed in the collection tube.

(7) 600 μL of a rinsing solution PW was added into the adsorption column CP3 and centrifuged at 12000 rpm for 30-60 s, and the waste liquid in in the collection tube was discarded. The adsorption column CP3 was placed into the collection tube.

(8) The step (7) was repeated.

(9) The adsorption column CP3 was placed in the collection tube and centrifuged at 12000 rpm for 2 min.

(10) The adsorption column CP3 was placed in a clean centrifuge tube, and 50-100 μL of an elution buffer solution EB were dropwise added on the middle portion of the adsorption membrane and left at room temperature for 2 min. The solution was centrifuged at 12000 rpm for 2 min, and the supernatant was taken and placed in a clean centrifuge tube to obtain a plasmid solution.

(11) 1 μl of the plasmid solution was taken and DEPC water was used as a blank control. The optical density values at the wavelengths of 260 nm and 280 nm were read on an ultraviolet spectrophotometer respectively.

## 1.9 Cell Culture

### 1.9.1 Resuscitation of 293T Cells

293T cell lines were taken out of a liquid nitrogen tank and immediately placed in a temperature-constant water bath kettle at 37 °C which is slightly shaken so as to rapidly thaw the cell lines. A cryopreservation tube, after being wiped with alcohol cotton, was transferred to a biological safety cabinet, and the cells were suction taken into a centrifuge tube loaded with a complete culture medium by a pipette, and centrifuged at 1000 rpm and 4 °C for 3 min. The supernatant was discarded, and 5 mL of the complete culture medium were added. The cells were uniformly dispersed by blowing with a pipette and then they were transferred to a 25 cm$^2$ culture flask. The cells were cultured in a 5% $CO_2$ cell incubator at 37 °C. After 24 h, the culture solution in the culture flask was replaced. According to growing states of the cells, the passage culture was conducted.

### 1.9.2 Passage of 293T cells

The cells were observed under a DMIL inverted phase-contrast microscope, and when the cells in the culture flask grown to 80 to 90%, the passage was conducted. The culture flask was removed from the culture incubator. The culture solution in the flask was discarded. 2 mL of a PBS buffer was added to rinse twice, and 500 μL of 0.25% pancreatin (containing 0.25% EDTA) was added to digest the cells for about 15 s until most of the cells bulge and become round. 1 mL of the complete medium was added to stop the digestion. The cell suspension was transferred into a centrifuge tube and centrifuged at 1000 rpm and 4 °C for 3 min. The supernatant was discarded. 4 mL of the complete medium was added to the centrifuge tube and gently blown and tapped until the cells were evenly dispersed, and then the cells were transferred to a new flask at 1:4 ratio.

### 1.9.3 Counting of 293T cell counts

A clear cell counting plate was taken and covered with a cover glass, and 10 μL of a uniformly dispersed cell suspension were added from the edge between the cover glass and the counting plate so as to fill the space between the counting plate and the cover glass. The plate was observed under an inverted microscope, to calculate the number of the cells in the four grid areas. For cells on the lines, only the cells on the left lines and on the upper lines were counted, and clustered cells are counted as a single cell.

The density of cells was calculated according to the equation:

$$\text{Cell numbers/mL} = (\text{cell numbers in four grid areas})/4 \times 10^4 \times \text{dilution time}$$

### 1.9.4 Formulation of medicinal solutions

2.5 mg of the inventive pharmaceutical compositions were precisely weighed into 2.5 ml of water to obtain 100 mg/ml of a standard stock solution of the inventive pharmaceutical compositions, and by the means of a gradient dilutions with DMEM culture medium, the sample solutions at 100 $\mu$g/ml, 10 $\mu$g/ml, 1 $\mu$g/ml, 0.1 $\mu$g/ml and 0.01 $\mu$g/ml were formulated.

### 1.10 Grouping and Detection

The detection on the cytotoxicity was conducted by grouping rates into a blank control group, and inventive pharmaceutical composition groups with five concentrations of 0.01 $\mu$g/mL, 0.1 kg/mL, 1 $\mu$g/mL, 10 $\mu$g/mL, and 100 $\mu$g/mL.

The detection on the anti-oxidative activity was conducted by grouping rats into a blank control group, a positive tBHQ medicinal group and inventive pharmaceutical composition groups with five concentrations of 0.01 $\mu$g/mL, 0.1 $\mu$g/mL, 1 $\mu$g/mL, 10 $\mu$g/mL, and 100 $\mu$g/mL.

The detection on the anti-inflammatory effects activity was conducted by grouping rats into a blank control group, a model group, a positive dexamethasone medicinal group, and inventive pharmaceutical composition groups with five concentrations of 0.01 $\mu$g/mL, 0.1 $\mu$g/mL, 1 $\mu$g/mL, 10 $\mu$g/mL, and 100 $\mu$g/mL.

### 1.10.1 Cytotoxicity Experiments

The experiment uses CCK-8 and LDH test kits to investigate impacts of the pharmaceutical compositions of the invention with different concentrations on the cytotoxicity of 293T cells.

The 293T cells in the logarithmic growth phase were taken and inoculated into a 96-well cell culture plate at a density of $2 \times 10^5$ cell/mL, and after growth along the walls to 70-80%, the inventive pharmaceutical compositions at 0.01-100 $\mu$g/mL were respectively added into the cell culture wells, each well with 100 $\mu$L and each concentration with 6 compound wells. Meanwhile, a control group without any medicaments was configured, with 3 repetitions. After 24 h cultures, the supernatant was suction taken and placed in the other new 96-well plate, and the LDH release amount was detected according to the operations of the LDH test kit. After the initial 96-well plate was washed by PBS, each well was added with 100 $\mu$L of a diluted 1 $\times$ CCK-8 working solution. The cells were incubated at 37 °C for 1 h. The absorbance (OD value) was measured by setting an enzyme-labelled meter at 450 nm.

According to the following equation, the cell survival rate was calculated:

$$\text{Cell survival rate (\%)} = (As\text{-}Ab)/(Ac\text{-}Ab) \times 100\%$$

where As represents the absorbance of experimental wells, Ab represents the absorbance of blank wells, and Ac represents the absorbance of control wells.

### 1.10.2 Transient co-transfections of 293T cells and measurements of ARE and NP$\kappa$B activities

### 1.10.2.1 Transient co-transfections of 293T cells

The 293T cells in the logarithmic growth phase were taken and inoculated into a 96-well cell culture plate at a density of $2 \times 10^5$ cell/mL, and after growth along the walls to 70-80%, a PEI (1 mg/ml) transfecting agent was used to simultaneously transfect them with ARE and NF-$\kappa$B luciferase reporter plasmids pGL4.37, pGL4.32 (100 ng/well) and a renilla luciferase reported plasmid pGL4.75 (10 ng/well), and they were cultured for 24 h.

### 1.10.2.2 Detections of ARE and NP$\kappa$B transcriptional activities with a dual-luciferase reporter system

After 24-h culture, the cells transfected with the ARE luciferase reporter plasmid pGL4.37 are added with tBHQ (10 $\mu$M) and safety concentrations of the sample solutions of the individual inventive pharmaceutical composition groups, and by setting a control group, and they were cultured 6 h; the cells transfected with NF-kB luciferase reporter plasmid pGL4.32 were respectively added with dexamethasone (10 $\mu$M) diluted by a TNF-$\alpha$ complete culture medium, and safety concentrations of the sample solutions of the individual inventive pharmaceutical composition groups, and by setting a control group, and a model group, and they were cultured for 6 h. The supernatant was discarded, and the cells were rinsed with PBS. After splitting, they were detected by using a Dual-Luciferase detection system. Each set of experiments were configured with 6 compound wells, with 3 repetitions. A relative luciferase activity value was obtained by comparing firefly luciferase activities and renilla luciferase activities. L/S = Luciferase activity value/Renilla activity value.

## 2 Experimental Results

### 2.1 Measurements of the cytotoxicity of the inventive pharmaceutical compositions on 293T cells

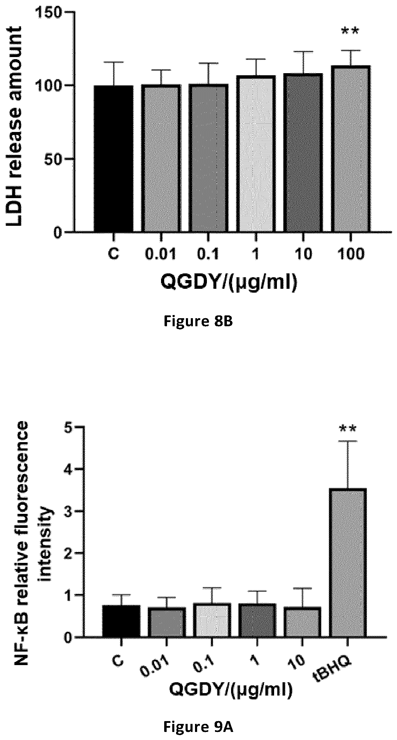

In comparison to the normal group, the 293T cell activities of the inventive pharmaceutical composition groups at 0.01-100 $\mu$g/ml did not have any significant differences, being medicinal safety concentrations for the 293T cells. The medicinal safety concentrations were used to discuss the antioxidative and anti-inflammatory effects of the pharmaceutical compositions of the invention (see Figs. 8A-8B).

### 2.2 Effects of the inventive pharmaceutical compositions on ARE reporter gene activities

293T cell transfections were used to verify the antioxidative activities of the sample solutions of t the inventive pharmaceutical compositions. The results showed that 1 $\mu$g/ml of the inventive pharmaceutical compositions may significantly induce the ARE luciferase activity (P<0.05) (see Table 12, Fig. 9A).

Table 12: Impacts of the inventive pharmaceutical compositions with different concentrations on the ARE luciferase activity of 293T cells (x $\pm$ SD) (n=18)

| Control | tBHQ | inventive pharmaceutical compositions ($\mu$g/ml) | | | |
|---|---|---|---|---|---|
| | | 0.01 | 0.1 | 1 | 10 |
| 0.76$\pm$0.25 | 3.55$\pm$1 12*** | 0.71$\pm$0.23 | 0.81$\pm$0.36 | 0.80$\pm$0.29 | 0.72$\pm$0.44 |
| Note: ***P<0.001 vs control | | | | | |

### 2.3 Impacts of the inventive pharmaceutical compositions on NF-kB reporter gene activities

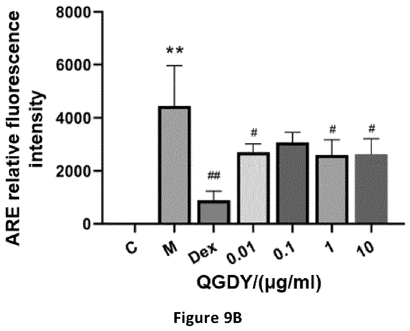

293T cells-transfected NF-kB promoter luciferase reporter plasmid was used to detect the anti-inflammatory activities of the inventive pharmaceutical compositions. The results showed that 0.01 $\mu$g/mL of the inventive pharmaceutical compositions can significantly inhibit the activity of TNF-$\alpha$-induced NF-kB promoter (P<0.05, P<0.01), indicating that: the inventive pharmaceutical compositions at concentrations of 0.01, 0.1 and 1 $\mu$g/mL each had better anti-inflammatory activities (see Table 13, Fig. 9B).

Table 13: Impacts of the inventive pharmaceutical compositions with different concentrations on the NF-$\kappa$B luciferase activity of 293T cells (x $\pm$ SD) (n=18)

| control | model | Dex | pharmaceutical compositions of the invention ($\mu$g/ml) | | | |
|---|---|---|---|---|---|---|
| | | | 0.01 | 0.1 | 1 | 10 |
| 17.46$\pm$4.29 | 4641.34$\pm$1 517.22*** | 968.61$\pm$3 48.54### | 2696.85$\pm$ 312.14# | 2912.02$\pm$374. 60 | 2618.26$\pm$582. 22# | 2598.75$\pm$58 1.63# |
| Note: ***P<0.001 vs control, ###P<0.001 vs model, #P<0.05 vs model. | | | | | | |

## 3. Experimental conclusions

The inventive pharmaceutical compositions do not have evident antioxidative effects, and the pharmaceutical compositions of the invention at 0.01-10 $\mu$g/ml can evidently inhibit the activity of TNF-$\alpha$ induced NF-kB, having significantly anti-inflammatory activity.

The invention and its embodiments have been described above generally and in detail. It will be appreciated by those skilled in the art that according to the teachings in the present application, various modifications, alternatives and other changes may be made directed to the details in the invention, and the technical solutions obtained by these changes each are in the scope of the invention.

**Claims**

1. A pharmaceutical composition, composed of the following traditional Chinese medicinal materials or extracts thereof in medicinal forms:

   Arctii Fructus, Belamcandae Rhizoma, Platycodonis Radix, Paeoniae Radix Rubra, Perillae folium, Lonicerae Japonicae Flos, Charred Hawthorn, Astragali Radix, Polygoni Cuspidati Rhizoma et Radix, Glycyrrhizae Radix et Rhizoma, and non-essential fermented tea, where measurement according to a high performance liquid chromatography as described in the specification, in each gram of the pharmaceutical composition, a content of arctiin is ≥4.4 mg, ≥5.5 mg, ≥7 mg or ≥7.3 mg.

2. A pharmaceutical composition, composed of the following traditional Chinese medicinal materials in medicinal forms by weight parts:

   2-4 parts by weight of Arctii Fructus,
   1-3 parts by weight of Belamcandae Rhizoma,
   1-3 parts by weight of Platycodonis Radi,
   1-3 parts by weight of Paeoniae Radix Rubra,
   1-3 parts by weight of Perillae folium,
   2-4 parts by weight of Lonicerae Japonicae Flos,
   1-3 parts by weight of Charred Hawthorn,
   2-4 parts by weight of Astragali Radix,
   2-4 parts by weight of Polygoni Cuspidati Rhizoma et Radix, and
   0.5-1.5 parts by weight of Glycyrrhizae Radix et Rhizoma;
   0.5-8 parts by weight of non-essential fermented tea.

3. The pharmaceutical composition according to claim 2, composed of the following traditional Chinese medicinal materials in medicinal forms by weight parts:

   3 parts by weight of Arctii Fructus,
   2 parts by weight of Belamcandae Rhizoma,
   2 parts by weight of Platycodonis Radi,
   2 parts by weight of Paeoniae Radix Rubra,
   2 parts by weight of Perillae folium,
   3 parts by weight of Lonicerae Japonicae Flos,
   2 parts by weight of Charred Hawthorn,
   3 parts by weight of Astragali Radix,
   3 parts by weight of Polygoni Cuspidati Rhizoma et Radix,
   1 part by weight of Glycyrrhizae Radix et Rhizoma, and
   3-5 parts by weight of non-essential fermented tea.

4. A pharmaceutical formulation comprising the pharmaceutical composition of any one of claims 1 to 3, and non-essential pharmaceutically acceptable adjuvants.

5. The pharmaceutical formulation according to claim 4, which is formulated in a unit dose form comprising 2-10 g, 3-5 g, 3 g or 4.5 g of the pharmaceutical composition.

6. The pharmaceutical formulation according to claim 4, which is a tea bag formulation.

7. The pharmaceutical formulation according to claim 6, wherein the traditional Chinese medicinal materials in medicinal forms are decoction piece powders of the traditional Chinese medicinal materials.

8. The pharmaceutical formulation according to claim 7, the decoction piece powders can pass through a Sieve No. 1; or the decoction piece powders can pass through the Sieve No. 1 but cannot pass through a Sieve No. 5.

9. Use of the pharmaceutical composition of any one of claims 1 to 3 in the manufacture of a medicament for uses described in one, more or all of the following items (1) to (4):

   (1) nourishing Qi, moistening lungs, clearing throats, relieving sore throats, and decontaminating,

(2) treating and/or preventing pharyngitis;
(3) eliminating phlegm; and
(4) relieving cough.

10. A method for achieving effects described in one, more or all of the following items (1)-(4) in a subject:

(1) nourishing Qi, moistening lungs, clearing throats, relieving sore throats, and decontaminating,
(2) treating and/or preventing pharyngitis;
(3) eliminating phlegm; and
(4) relieving cough;

the method comprising administering to the subject with the pharmaceutical composition of any one of claims 1 to 3 or the pharmaceutical formulation of any one of claims 4 to 8.

← Solvent front

← Baseline

① ② ③

Figure 1A

← Solvent front

← Baseline

① ② ③ ④

Figure 1B

← Solvent front

← Baseline

① ②

**Figure 1C**

**Figure 2A**          **Figure 2B**

**Figure 2C**          **Figure 2D**

**Figure 3**

Figure 4

Figure 5

Figure 6A

Figure 6B

**Figure 7A**

**Figure 7B**

**Figure 7C**

**Figure 7D**

Figure 7E

Figure 8A

Figure 8B

Figure 9A

Figure 9B

**EP 4 215 203 A1**

## INTERNATIONAL SEARCH REPORT

| International application No. |
|---|
| **PCT/CN2021/102174** |

**A.  CLASSIFICATION OF SUBJECT MATTER**

A61K 36/88(2006.01)i；  A61K 36/734(2006.01)i；  A61K 36/704(2006.01)i；  A61K 36/535(2006.01)i；  A61K 36/484(2006.01)i；  A61K 36/481(2006.01)i；  A61K 36/35(2006.01)i；  A61K 36/34(2006.01)i；  A61K 36/28(2006.01)i；  A61K 36/71(2006.01)i；  A61P 11/04(2006.01)i；  A61P 31/14(2006.01)i；  A61P 11/10(2006.01)i；  A61P 11/14(2006.01)i

According to International Patent Classification (IPC) or to both national classification and IPC

**B.  FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS, CNTXT, VEN, DWPI, SIPOABS, CJFD, CNKI, MEDNPL, 超星读秀, 万方, 中国药物专利数据库, 《国家新药注册数据》（1985-2000）, E药全库, 必应学术, 百度, ISI Web of Science: 关键词: 牛蒡子, 鼠粘子, 大力子, 蝙蝠刺, 牛子, 鼠粘草, 射干, 野萱花, 扁竹, 谢干, 寸干, 桔梗, 赤芍, 木芍药, 红芍药, 草芍药, 山芍药, 卵叶芍药, 野芍药, 金银花, 忍冬花, 银花, 苏花, 金花, 金藤花, 双花, 双苞花, 二花, 二宝花, 山楂, 鼠查, 山里红, 酸查, 楂肉, 焦楂, 红果, 山渣, 山查, 黄芪, 黄耆, 绵芪, 口芪, 北芪, 生芪, 元芪, 虎杖, 大虫杖, 苦杖, 酸杖, 斑杖, 斑根, 酸榴根, 土地榆, 酸通, 雄黄连, 大活血, 血藤, 紫金龙, 黄地榆, 红贯脚, 山茄子, 虎仗, 甘草, 生草, 炙草, 紫苏叶, 苏叶, 发酵茶, 袋泡茶, 茶, 红茶, 乌龙茶, 粉碎, 益气润肺, 咽炎, 祛痰, 止咳 Great Burdock, Fructus Arctii, Arctium lappa, Blackberrylily Rhizome, Rhizoma Belamcandae, Platycodon Root, Radix Platycodi, Radix Platycodonis, Red Paeony Root, Radix Paeoniae Rubra, Flos Lonicerae, Honeysuckle, Hawthorn Fruit, fruit of chinese Hawthorn, fruit of Large chinese Hawthorn, fruit of Nippon Hawthorn, Fruit of Hupeh hawthon, Fruit of Redhaw Hawthorn, fruit of Yannan hawthorn, Fructus Crataegi, Crataegus, Milkvetch Root, Radix Astragali, Rhizoma Polygoni Cuspidati, Giant Knotweed Rhizome, Giant Knotweed, Japanese Fleeceflower, Liquorice Root, Radix Glycyrrhizae, purple perilla, yeast tea, tea bag, tea, black tea, oolong tea, smash, supplementing qi and moistening lung, pharyngitis, eliminating phlegm, cough

**C.  DOCUMENTS CONSIDERED TO BE RELEVANT**

| Category* | Citation of document, with indication, where appropriate, of the relevant passages | Relevant to claim No. |
|---|---|---|
| PX | CN 111991484 A (TIANJIN UNIVERSITY OF TRADITIONAL CHINESE MEDICINE) 27 November 2020 (2020-11-27) <br> claims 1-8 | 1-10 |
| A | CN 111298009 A (HUBEI WUDANG BIOPHARMACEUTICAL SCIENCE AND TECHNOLOGY CO., LTD.) 19 June 2020 (2020-06-19) <br> entire document | 1-10 |

☑ Further documents are listed in the continuation of Box C.       ☑ See patent family annex.

| | |
|---|---|
| *    Special categories of cited documents: <br> "A"  document defining the general state of the art which is not considered to be of particular relevance <br> "E"  earlier application or patent but published on or after the international filing date <br> "L"  document which may throw doubts on priority claim(s) or which is cited to establish the publication date of another citation or other special reason (as specified) <br> "O"  document referring to an oral disclosure, use, exhibition or other means <br> "P"  document published prior to the international filing date but later than the priority date claimed | "T"  later document published after the international filing date or priority date and not in conflict with the application but cited to understand the principle or theory underlying the invention <br> "X"  document of particular relevance; the claimed invention cannot be considered novel or cannot be considered to involve an inventive step when the document is taken alone <br> "Y"  document of particular relevance; the claimed invention cannot be considered to involve an inventive step when the document is combined with one or more other such documents, such combination being obvious to a person skilled in the art <br> "&"  document member of the same patent family |

| Date of the actual completion of the international search | Date of mailing of the international search report |
|---|---|
| **03 September 2021** | **28 September 2021** |

| Name and mailing address of the ISA/CN | Authorized officer |
|---|---|
| **China National Intellectual Property Administration (ISA/CN)** <br> **No. 6, Xitucheng Road, Jimenqiao, Haidian District, Beijing 100088** <br> **China** | |
| Facsimile No. **(86-10)62019451** | Telephone No. |

Form PCT/ISA/210 (second sheet) (January 2015)

# EP 4 215 203 A1

<table>
<tr><td colspan="2" style="text-align:center"><strong>INTERNATIONAL SEARCH REPORT</strong></td><td>International application No.<br><strong>PCT/CN2021/102174</strong></td></tr>
</table>

**C.    DOCUMENTS CONSIDERED TO BE RELEVANT**

| Category* | Citation of document, with indication, where appropriate, of the relevant passages | Relevant to claim No. |
|---|---|---|
| A | CN 102091191 A (LU, Zhengping) 15 June 2011 (2011-06-15)<br>entire document | 1-10 |
| A | 沈健 等 (SHEN, Jian et al.). "内外合治治疗小儿反复呼吸道感染风热咳嗽的疗效研究 (non-official translation: Study on the Effect of Combined Internal and External Treatment on Children With Recurrent Respiratory Tract Infections and Wind-Heat Cough)"<br>临床合理用药 *(Chinese Journal of Clinical Rational Drug Use)*,<br>Vol. 7, No. 4, 30 April 2014 (2014-04-30),<br>pages 121-122, section 1.6 treatment methods | 1-10 |
| A | 吕昌群 (LYU, Changqun). "山楂射干饮治疗儿童急性扁桃体炎60例 (non-official translation: 60 Children with Acute Tonsilittis Treated with a Therapeutic Drink Made of Belamcanda chinensis and Crateagus Pinnatifida)"<br>河南中医 *(Henan Traditional Chinese Medicine)*,<br>Vol. 30, No. 1, 31 January 2010 (2010-01-31),<br>pp. 64-65 | 1-10 |

Form PCT/ISA/210 (second sheet) (January 2015)

**INTERNATIONAL SEARCH REPORT**

International application No.

**PCT/CN2021/102174**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a.  ☑  forming part of the international application as filed:

        ☑  in the form of an Annex C/ST.25 text file.

        ☐  on paper or in the form of an image file.

    b.  ☐  furnished together with the international application under PCT Rule 13*ter*.1(a) for the purposes of international search only in the form of an Annex C/ST.25 text file.

    c.  ☐  furnished subsequent to the international filing date for the purposes of international search only:

        ☐  in the form of an Annex C/ST.25 text file (Rule 13*ter*.1(a)).

        ☐  on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

**INTERNATIONAL SEARCH REPORT**

International application No.

**PCT/CN2021/102174**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **10**
   because they relate to subject matter not required to be searched by this Authority, namely:

   [1] Claim 10 relates to a method of disease treatment. Therefore, the search and written opinion are provided based on the reasonable expectation of claim 10 to be a corresponding pharmaceutical use claim.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

INTERNATIONAL SEARCH REPORT
Information on patent family members

International application No.

**PCT/CN2021/102174**

| Patent document cited in search report | | | Publication date (day/month/year) | Patent family member(s) | Publication date (day/month/year) |
|---|---|---|---|---|---|
| CN | 111991484 | A | 27 November 2020 | None | |
| CN | 111298009 | A | 19 June 2020 | None | |
| CN | 102091191 | A | 15 June 2011 | None | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Standards for Preparation of Animal Models Suffered from Acute Pharyngitis. Traditional Chinese Medicine Pharmacology and Clinics. Traditional Chinese medicine Experimental Pharmacology Professional Committee of Traditional Chinese Medicine Institute, 2018, vol. 34